# EUROPEAN PATENT SPECIFICATION

(11) **EP 4 599 755 B1**
(45) Date of publication and mention of the grant of the patent: **09.09.2026**
(21) Application number: 24760652.8
(22) Date of filing: 16.02.2024
(51) Int. Cl.: A61B 5/00, A61B 5/024, A61B 5/01, G04G 21/02, G04G 17/08, G06F 1/16

(54) **WEARABLE ELECTRONIC DEVICE COMPRISING SENSOR MODULE**
TRAGBARE ELEKTRONISCHE VORRICHTUNG MIT SENSORMODUL
DISPOSITIF ÉLECTRONIQUE À PORTER SUR SOI COMPRENANT UN MODULE DE CAPTEUR

(30) Priority: 24.02.2023 KR 20230025332; 05.04.2023 KR 20230044838
(43) Date of publication of application: 13.08.2025
(73) Proprietor: Samsung Electronics Co., Ltd., Suwon-si, Gyeonggi-do 16677 (KR)
(72) Inventor: JEONG, Injo, Suwon-si Gyeonggi-do 16677 (KR); BAEK, Sanghyun, Suwon-si Gyeonggi-do 16677 (KR); YOO, Hyunguk, Suwon-si Gyeonggi-do 16677 (KR); LEE, Jeahyuck, Suwon-si Gyeonggi-do 16677 (KR); KIM, Younghyun, Suwon-si Gyeonggi-do 16677 (KR); PARK, Minho, Suwon-si Gyeonggi-do 16677 (KR); JO, Seongwook, Suwon-si Gyeonggi-do 16677 (KR)
(74) Representative: Gulde & Partner
(86) International application number: PCT/KR2024/095347
(87) International publication number: WO 2024/177464

(56) References cited:
- WO-A1-2022/127628
- KR-A- 20200 093 247
- KR-A- 20200 100 487
- KR-A- 20210 148 267
- KR-A- 20220 117 613
- KR-A- 20220 129 033
- US-A1- 2021 093 237
- US-A1- 2021 290 120
- US-A1- 2023 032 169

## Description

### [Technical Field]

Embodiments disclosed herein relate to a wearable electronic device including a sensor module.

### [Background Art]

In line with development of electronic, information, or communication technologies, various functions tend to be integrated into a single electronic device. Electronic devices now have a high level of performance and compactness and thus are carried and used in daily life, and various types of portable electronic devices have been provided. For example, multiple portable electronic devices such as smartphones, tablet PCs, smart watches, wireless earphones, and/or smart glasses can be carried and used by users.

Various types of wearable electronic devices, such as glasses or watches, have been disclosed such that the electronic devices can be worn on human bodies and used. For example, a wearable electronic device, which is worn on a human body, may provide the user's biometric information by using sensors. For example, wearable electronic devices may include sensors capable of acquiring biometric information such as the user's body temperature, electrocardiogram (ECG), breathing, electromyogram (EMG), electrooculogram (EOG), electroencephalogram (EEG), blood glucose, SpO₂, photoplethysmogram (PPG), or body temperature, and various pieces of other information.

Such a wearable electronic devices may include a rear plate formed such that sensors for acquiring biometric information are protected, and light or heat can be transferred to the sensors. For example, the rear plate may include an at least partially transparent or opaque area, and may be configured such that reflected light or conductive heat for acquiring biometric information can be transferred to internal sensors.

The above-described information may be provided as a background technology intended to help understanding of the disclosure herein. No claim or determination is raised regarding whether any of the above description is applicable as a prior art in connection with the disclosure herein.

US 2021/093237 A1 relates to a wearable physiological monitoring device, such as a watch, for measuring oxygen saturation. It features a back cover with windows for light emitters and detectors. To improve accuracy and prevent optical crosstalk, the device incorporates optical barriers. These barriers, which can be optically opaque ink coatings, are applied to the side surfaces of the window openings.

US 2021/290120 A1 relates to a wearable health monitoring device featuring an optical sensor with a convex, skin-facing cover. This convex curvature is designed to improve optical coupling by applying pressure and ensuring firm contact with the wearer's skin. To enhance signal quality, the sensor includes an internal light barrier construct that forms walls of chambers, physically separating the emitters and detectors to prevent crosstalk.

WO 2022/127628 A1 discloses a wearable electronic device, such as a smartwatch, focused on improving the accuracy of body temperature measurement. To achieve this, it proposes using a heat-insulating structure to shield the temperature sensor from heat generated by internal electronic components. Additionally, it uses a heat-conducting sheet or plate to create an efficient thermal path from the user's skin to the sensor.

US 2023/032169 A1 discloses a sensor structure for a wearable device designed to perform both biometric and thermal sensing. It features a dual-purpose electrode system that measures bioelectrical signals (e.g., ECG) while also serving as a thermal conduction path to transfer body heat to an internal temperature sensor. The document explicitly discloses a stacked, multi-board PCB configuration to house these components.

### [Detailed Description of the Invention]

### [Technical Solution]

The present invention is directed to subject matter as defined in the claims.

### [Brief Description of Drawings]

In relation to an embodiment of the disclosure, an aspect or other aspects, configurations, and/or advantages thereof described above may become clearer through the following detailed descriptions referring to the accompanying drawings.
FIG. 1 is a block diagram of an electronic device in a network environment according to an embodiment disclosed in the document.
FIG. 2 is a front perspective view of a wearable electronic device according to an embodiment of the disclosure.
FIG. 3 is a rear perspective view of a wearable electronic device according to an embodiment of the disclosure.
FIG. 4 is an exploded perspective view showing a wearable electronic device according to an embodiment of the disclosure.
FIG. 5 is a perspective view of a rear plate of a wearable electronic device according to an embodiment of the disclosure.
FIG. 6 is a side view of a rear plate of a wearable electronic device according to an embodiment of the disclosure.
FIG. 7A is a perspective view of a second rear plate of a wearable electronic device according to an embodiment of the disclosure.
FIG. 7B is a cross-sectional view taken along line B-B' in FIG. 7A.
FIG. 8 is a perspective view of a rear plate of a wearable electronic device according to an embodiment of the disclosure.
FIG. 9 is a cross-sectional view taken along line C-C' in FIG. 8.
FIG. 10 is a cross-sectional perspective view of a wearable electronic device according to an embodiment of the disclosure.
FIG. 11 is a cross-sectional view of a wearable electronic device according to an embodiment of the disclosure.
FIG. 12 is a perspective view of a state in which a second rear plate of a wearable electronic device is removed according to an embodiment of the disclosure.
FIG. 13 is a partial cross-sectional view of a wearable electronic device according to an embodiment of the disclosure.
FIG. 14 is a plan view of a bridge connector of a wearable electronic device according to an embodiment of the disclosure.
FIG. 15A is a perspective view of a rear plate of a wearable electronic device according to an embodiment of the disclosure.
FIG. 15B is a perspective view of a rear plate of a wearable electronic device according to an embodiment of the disclosure.
FIG. 15C is a perspective view of a rear plate of a wearable electronic device according to an embodiment of the disclosure.
FIG. 16A is a plan view of a rear plate of a wearable electronic device according to an embodiment of the disclosure.
FIG. 16B is a plan view of a rear plate of a wearable electronic device according to an embodiment of the disclosure.
FIG. 16C is a plan view of a rear plate of a wearable electronic device according to an embodiment of the disclosure.
FIG. 17 is a plan view of a rear plate of a wearable electronic device according to an embodiment of the disclosure.
FIG. 18A is a plan view of a rear plate of a wearable electronic device according to an embodiment of the disclosure.
FIG. 18B is a plan view of a rear plate of a wearable electronic device according to an embodiment of the disclosure.
FIG. 18C is a plan view of a rear plate of a wearable electronic device according to an embodiment of the disclosure.
FIG. 18D is a plan view of a rear plate of a wearable electronic device according to an embodiment of the disclosure.
FIG. 18E is a plan view of a rear plate of a wearable electronic device according to an embodiment of the disclosure.
FIG. 19A is a plan view of a rear plate of a wearable electronic device according to an embodiment of the disclosure.
FIG. 19B is a cross-sectional perspective view taken along line D-D' in FIG. 19A.
FIG. 20A is a plan view of a rear plate of a wearable electronic device according to an embodiment of the disclosure.
FIG. 20B is a cross-sectional perspective view taken along line E-E' in FIG. 20A.
FIG. 21 is a plan view of a rear plate of a wearable electronic device according to an embodiment of the disclosure.
FIG. 22A is a plan view of a rear plate of a wearable electronic device according to an embodiment of the disclosure.
FIG. 22B is a plan view of a rear plate of a wearable electronic device according to an embodiment of the disclosure.
FIG. 23A is a plan view of a rear plate of a wearable electronic device according to an embodiment of the disclosure.
FIG. 23B is a plan view of a rear plate of a wearable electronic device according to an embodiment of the disclosure.
FIG. 24 is a flowchart of a method for manufacturing a rear plate of an electronic device according to an embodiment of the disclosure.
FIG. 25 is a perspective view of a rear plate of a wearable electronic device according to an embodiment of the disclosure.
FIG. 26 is a cross-sectional view taken along line F-F' in FIG. 8.
FIG. 27 is a flow diagram illustrating a method of assembling a wearable electronic device according to an embodiment of the disclosure.

Throughout the accompanying drawings, similar reference numerals may be assigned to similar components, configurations, and/or structures.

### [Mode for Carrying out the Invention]

Hereinafter, in order to enable the disclosure to be easily implemented by a person skilled in the art in the technical field belonging to the disclosure, embodiments of the disclosure will be described in detail with reference to the drawings. However, the disclosure may be implemented in various other types and is not limited to the embodiments disclosed herein. In relation to the descriptions of drawings, identical or similar reference signs may be used to refer to identical or similar elements. In addition, in relation to drawings or the descriptions related thereto, descriptions of well-known functions and configurations may be omitted for clarity and conciseness. In the following description, although numerous features may be designated as optional, it is nevertheless acknowledged that all features defined in claim 1 are not to be read as optional.

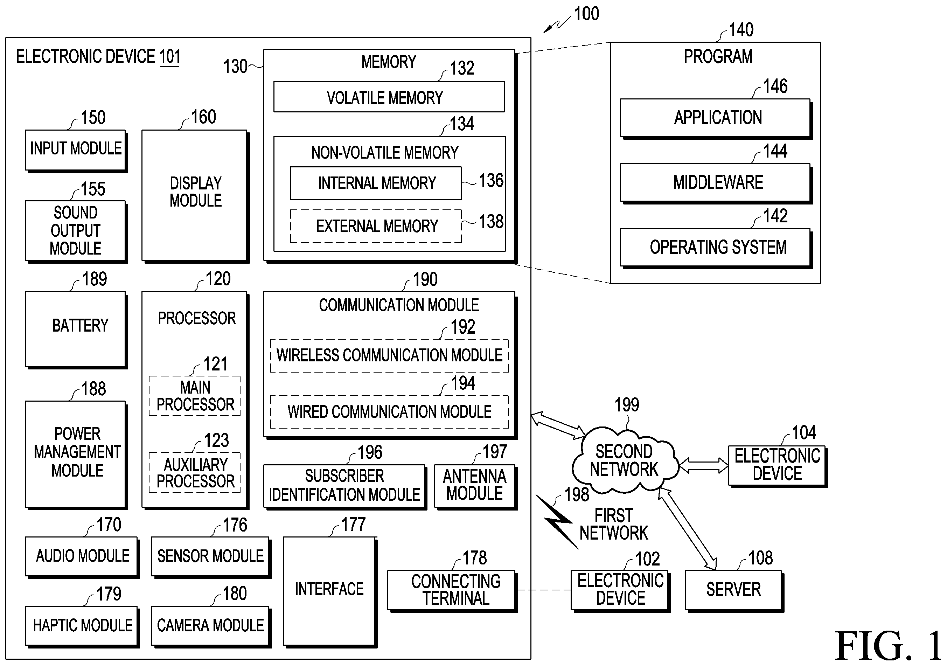

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or loT-related technology.

In the following detailed descriptions, a longitudinal direction, a width direction, and/or a thickness direction of an electronic device may be mentioned, the longitudinal direction may be defined as the "Y-axis direction", the width direction may be defined as the "X-axis direction", and/or the thickness direction may be defined as the "Z-axis direction". In an embodiment, in relation to the direction in which an element is oriented, in addition to the rectangular coordinate system illustrated in drawings, a 'negative/positive (-/+)" may be mentioned together therewith. For example, the front surface of an electronic device or a housing may be defined as "a surface oriented in the +Z-axis direction", and the rear surface thereof may be defined as "a surface oriented the -Z-axis direction". In an embodiment, the side surface of an electronic device or a housing may include an area oriented in the +X-axis direction, an area oriented in the +Y-axis direction, an area oriented in the -X-axis direction, and/or an area oriented in the -Y-axis direction. In another embodiment, the 'X-axis direction' may be a meaning including both the "-X-axis direction' and the '+X-axis direction'. The foregoing is based on the rectangular coordinate system illustrated in drawings for the sake of brevity of descriptions, and it should be noted that the directions or the descriptions of elements do not limit the embodiments disclosed in the disclosure. For example, the aforementioned direction, in which the front surface or the rear surface is oriented, may be changed according to an unfolded state or folded state of an electronic device, and the aforementioned directions may be differently interpreted according to a gripping habit of a user.

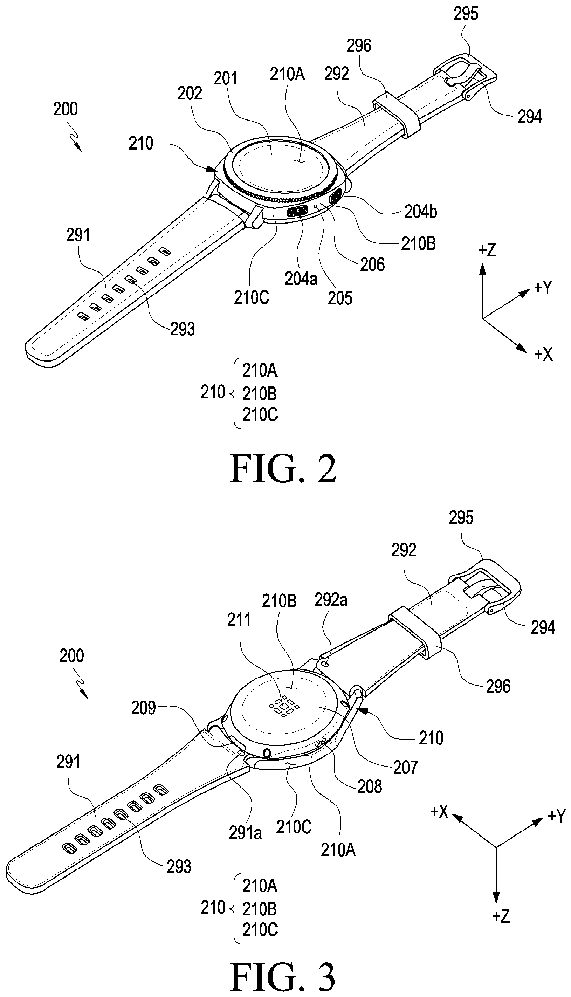

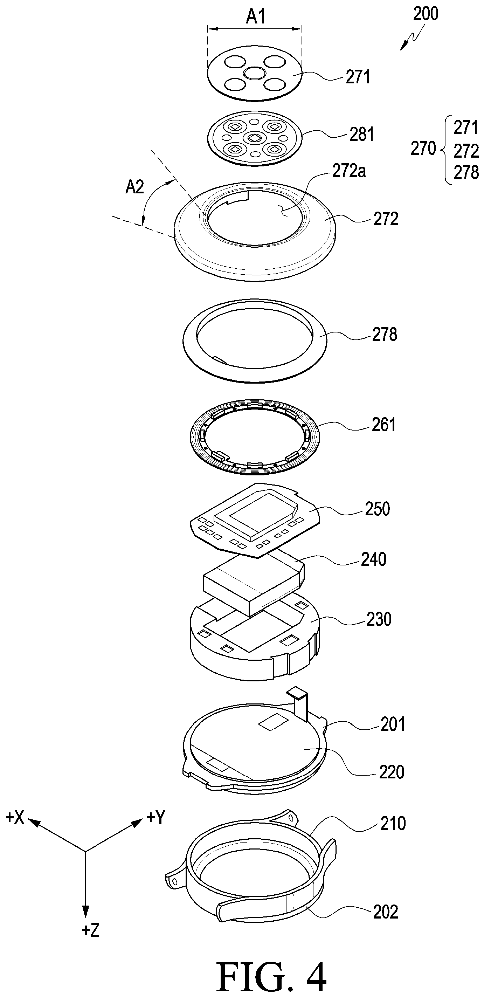

The configurations of a wearable electronic device 200 in FIG. 2 and FIG. 4 may be entirely or partially the same as the configurations of the electronic device (or wearable electronic device)101 in FIG. 1.

FIG. 2 is a front perspective view showing a wearable electronic device according to various embodiments disclosed in the document. FIG. 3 is a rear perspective view showing the wearable electronic device in FIG. 2.

In the following detailed descriptions, in FIG. 2 to FIG. 4, the width direction or the longitudinal direction of a wearable electronic device 200 or a housing 210 (or a side bezel structure) may be any one of the "X-axis direction" and the "Y-axis direction" of the illustrated rectangular coordinate system. In case it is necessary to distinguish the width direction or the longitudinal direction, the directions of the rectangular coordinate system illustrated in drawings may be illustrated therewith. In the rectangular coordinate system in FIG. 2 to FIG. 4, the "Z-axis direction" may mean the thickness direction of the wearable electronic device 200 or the housing 210. In an embodiment, the direction, in which the front surface (e.g., the first surface 210A in FIG. 2) of the wearable electronic device 200 or the housing 210 is oriented, may be defined as a "first direction" or the "+Z-axis direction", and the direction, in which the rear surface (e.g., the second surface 210B in FIG. 3) of the wearable electronic device 200 or the housing 210 is oriented, may be defined as a "second direction" or the "-Z-axis direction".

Referring to FIG. 2 and FIG. 3, the wearable electronic device 200 according to an embodiment may include the housing 210 including a first surface (or a front surface) 210A, a second surface (or a rear surface) 210B, and a side surface 210C for surrounding a space between the first surface 210A and the second surface 210B, and wearable members 291 and 292 connected to at least a part of the housing 210 and configured to allow the wearable electronic device 200 or the housing 210 to be worn on a part (e.g., a wrist or an ankle) of the body of a user. In another embodiment (not shown), the housing 210 may also be referred to as a structure forming a part of the first surface 210A, the second surface 210B, and the side surface 210C in FIG. 2. According to an embodiment, the first surface 210A may be formed by a front plate 201 (e.g., a polymer plate or a glass plate including various coating layers) of which at least a portion is substantially transparent. The second surface 210B may be formed by a rear plate 207, at least a part of which is configured as a substantially transparent and/or opaque area. In an embodiment, in case an electronic device includes a first sensor module 211 disposed on the second surface 210B, the rear plate 207 may include an area (e.g., the first rear plate 271 in FIG. 4) which is at least partially transparent. For example, the rear plate 207 may be formed by coated or colored glass, ceramic, polymer, metal (e.g., aluminum, stainless steel (STS), or magnesium), or a combination of at least two of the materials. The side surface 210C may be coupled to the front plate 201 and/or the rear plate 207, and may be formed by a side bezel structure (or a "lateral member") 206 including metal and/or polymer. In an embodiment, the rear plate 207 and the side bezel structure 206 may be integrally formed, and may include an identical material (e.g., a metal material such as aluminum). The wearable members 291 and 292 may be formed of various materials and in various forms. Integrated links and multiple unit links may be formed to be movable with respect to each other by woven fabrics, leather, rubber, urethane, metal, ceramic, or a combination of at least two of the materials.

According to an embodiment, the wearable electronic device 200 may include at least one of a display (220, see FIG. 4) (e.g., the display module 160 in FIG. 1), audio holes 205 and 208, a first sensor module 211 (e.g., the sensor module 176 in FIG. 1), key input devices 202, 204a, and 204b, and a connector hole 209. In an embodiment, at least one (e.g., the key input devices 202, 204a, or 204b, the connector hole 209, or the first sensor module 211) of these elements may be omitted from the wearable electronic device 200, or other elements may be additionally included therein.

For example, a display (e.g., the display 220 in FIG. 4) may be exposed through a significant portion of the front plate 201. The shape of the display 220 may be a shape corresponding to the shape of the front plate 201, and may have various shapes such as a circle, an ellipse, or a polygon. The display 220 may be coupled to or disposed adjacent to a touch detection circuit, a pressure sensor capable of measuring the intensity (pressure) of touch, and/or a fingerprint sensor.

The audio holes 205 and 208 may include a microphone hole 205 and a speaker hole 208. A microphone for obtaining external sound may be disposed in the microphone hole 205, and according to an embodiment, multiple microphones may be arranged to be able to detect the direction of sound. The speaker hole 208 may be used as an external speaker and a receiver for a phone call. According to an embodiment, a speaker (e.g., a piezo speaker) may be included therein without a speaker hole.

A sensor module (e.g., the sensor module 176 in FIG. 1) may generate an electrical signal or a data value corresponding to an internal operation state of the wearable electronic device 200 or an external environmental state. In an embodiment, the sensor module may include a first sensor module 211, which is a biometric sensor (e.g., an HRM sensor), disposed on the second surface 210B of the housing 210. The wearable electronic device 200 may further include a sensor module not illustrated, for example, at least one of a gesture sensor, a gyro sensor, a barometric pressure sensor, a magnetic sensor, an acceleration sensor, a grip sensor, a color sensor, an infrared (IR) sensor, a biometric sensor, a temperature sensor, a humidity sensor, or an illuminance sensor.

The key input devices 202, 204a, and 204b may include a wheel key 202 which is disposed on the first surface 210A of the housing 210 and is rotatable in at least one direction, and/or side key buttons 204a and 204b arranged on the side surface 210C of the housing 210. The wheel key 202 may have a shape corresponding to the shape of the front plate 201. In another embodiment, the wearable electronic device 200 may not include all or a part of the key input devices 202, 204a, and 204b mentioned above, and the key input device 202, 240a, or 204b not included therein may be implemented as a different type such as a soft key, on the display 220. The connector hole 209 may include other connector holes (not shown) capable of accommodating a connector (for example, a USB connector) for transmitting or receiving power and/or data to or from an external electronic device, and capable of accommodating a connector for transmitting or receiving an audio signal to or from an external electronic device. For example, the wearable electronic device 200 may further include a connector cover (not shown) for covering at least a part of the connector hole 209 and blocking external foreign materials from being introduced through the connector hole.

The wearable members 291 and 292 may be detachably fastened to at least a partial area of the housing 210 by using a locking member 291a and 292a. For example, the locking member 291a and 292a may include a fastening component such as a POGO pin, and according to an embodiment, may be replaced with a protrusion(s) or a recess(es) formed on the wearable members 291 and 292. For example, the wearable members 291 and 292 may be coupled in a manner of being engaged with a recess or a protrusion formed on the housing 210. The wearable members 291 and 292 may include one or more of a fixation member 294, a fixation member fastening hole 293, a band guide member 295, and a band fixation ring 296.

The fixation member 294 may be configured to fix the housing 210 and the wearable members 291 and 292 to a part (e.g., a wrist or an ankle) of the body of a user. The fixation member fastening hole 293 may be formed to correspond to the fixation member 294 so that the housing 210 and the wearable members 291 and 292 are fixed to a part of the body of the user. The band guide member 295 may be configured to limit a motion range of the fixation member 294 in case the fixation member 294 is fastened to the fixation member fastening hole 293, and thus the wearable members 291 and 292 may be fastened to a part of the body of a user in a state of being in close contact therewith. The band fixation ring 296 may limit a motion range of the wearable members 291 and 292 in a state where the fixation member 294 and the fixation member fastening hole 293 are fastened to each other.

In FIG. 4, the illustration of the wearable members 291 and 292 in FIG. 2 and FIG. 3 may be omitted. Referring to FIG. 4, the wearable electronic device 200 may include a housing 210 (or a side bezel structure), a wheel key 202, a front plate 201 (e.g., the front plate 201 in FIG. 2), a display 220, a first antenna, a second antenna (e.g., a coil assembly 261), a support member 230 (e.g., a bracket), a battery 240, a printed circuit board 250 (e.g., a main circuit board), a sealing member (not shown), a rear plate 270 (e.g., the rear plate 207 in FIG. 3), and wearable members (e.g., the wearable members 291 and 292 in FIG. 2 or FIG. 3). At least one of elements of the wearable electronic device 200 may be the same as or similar to at least one of elements of the wearable electronic device 200 in FIG. 2 or FIG. 3, and hereinafter, overlapping descriptions thereof will be omitted. The support member 230 may be disposed inside the wearable electronic device 200 and connected to the housing 210, or may be integrally formed with the housing 210. For example, the support member 230 may be formed of a metal material and/or a non-metal (e.g., polymer) material. The support member 230 may have a surface to which the display 220 is coupled, and the other surface to which the printed circuit board 250 is coupled. A processor (e.g., the processor 120 in FIG. 1), memory (e.g., the memory 130 in FIG. 1), and/or an interface (e.g., the interface 177 in FIG. 1) may be mounted on the printed circuit board 250. For example, the processor may include one or more of a central processing unit, an application processor, a graphic processing unit (GPU), a sensor processor, and a communication processor.

For example, the memory may include volatile memory (e.g., the volatile memory 132 in FIG. 1) or non-volatile memory (e.g., the non-volatile memory 134 in FIG. 1). For example, the interface may include a high-definition multimedia interface (HDMI), a universal serial bus (USB) interface, an SD card interface, and/or an audio interface. For example, the interface may electrically or physically connect the wearable electronic device 200 to an external electronic device, and may include a USB connector, an SD card/MMC connector, or an audio connector.

The battery 240 may be a device for supplying power to at least one element of the wearable electronic device 200, and for example, may include a non-rechargeable primary cell, a rechargeable secondary cell, or a fuel cell. For example, at least a part of the battery 240 may be disposed on substantially the same plane as the printed circuit board 250. The battery 240 may also be integrally disposed inside the wearable electronic device 200, or may also be disposed to be detachable/attachable from/to the wearable electronic device 200.

In case the housing 210 or the support member 230 includes a metal material, for example, the first antenna may use at least a portion of the housing 210 and/or the support member 230 as a radiation conductor. For example, a processor (e.g., the processor 120 in FIG. 1) or a communication module (e.g., the communication module 190 in FIG. 1) may be configured to perform wireless communication by using at least a portion of the housing 210 and/or the support member 230. In an embodiment, the first antenna (not shown) may be disposed between the display 220 and the support member 230. For example, the first antenna may include a near field communication (NFC) antenna, a wireless charging antenna, and/or a magnetic secure transmission (MST) antenna. For example, the first antenna may perform short-range communication with an external device or may wirelessly transmit and receive power required for charging, and may transmit a short-range communication signal or a magnetic-based signal including payment data. In an embodiment, an antenna structure may be formed by a part of the housing 210 and/or the support member 230, or a combination thereof, and an antenna structure not illustrated, which is disposed between the display 220 and the support member 230, may be formed. In an embodiment, the antenna structure by the housing 210 and/or the support member 230 and the antenna structure not illustrated, which is disposed between the display 220 and the support member 230, may be used for wireless communication functions according to different communication protocols.

An auxiliary circuit board 281 may be disposed in a space surrounded by the housing 210, between the printed circuit board 250 and the rear plate 270. The auxiliary circuit board 281 may include a second antenna, for example, a near field communication (NFC) antenna, a wireless charging antenna, and/or a magnetic secure transmission (MST) antenna. In an embodiment disclosed in the document, the second antenna may be implemented as a coil assembly 261 separated from the auxiliary circuit board 281. For example, the printed circuit board 250 and/or the auxiliary circuit board 281, by using the second antenna, may perform short-range communication with an external device or may wirelessly transmit and receive power required for charging, and may transmit a short-range communication signal or a magnetic-based signal including payment data. In an embodiment, an antenna structure may be formed by a part of the housing 210 and/or the rear plate 270 or a combination thereof. In an embodiment, in case the wearable electronic device 200 (e.g., the wearable electronic device 200 in FIG. 2 and FIG. 3) includes a sensor module (e.g., the sensor module 176 in FIG. 1 or the sensor module 211 in FIG. 3), a sensor circuit disposed on the auxiliary circuit board 281 or a sensor element separated from the auxiliary circuit board 281 may be disposed thereon. For example, the sensor circuit or the sensor element may include a light-emitting element, a photoelectric conversion element, or an electrode pad. For example, an electronic component (e.g., the auxiliary circuit board 281 in FIG. 4) provided as the sensor module 211 may be disposed between the printed circuit board 250 and the rear plate 270.

According to an embodiment, the rear plate 270 may include a first rear plate 271 and a second rear plate 272 disposed to surround at least a part of the first rear plate 271. In an embodiment, when seen in the Z-axis direction, the second rear plate 272 may have a loop shape forming or defining a seat opening 272a, and the first rear plate 271 may be disposed in the seat opening 272a of the second rear plate 272. In the illustrated embodiment, the second rear plate 272 is illustrated in a substantially circular loop shape when seen in the Z-axis direction, but is not limited thereto, and the second rear plate 272 may have a polygonal loop shape. In an embodiment, the first rear plate 271 may be coupled to the second rear plate 272 by a sealing member or an adhesive member such as double-sided tape, and for example, in the seat opening 272a, a sealing structure or a waterproof structure may be formed between the first rear plate 271 and the second rear plate 272. However, according to an embodiment, the first rear plate 271 and the second rear plate 272 may be formed or manufactured as a single plate member without being coupled to each other after being separately manufactured. In this case, the "first rear plate" may be referred to as a single plate member including the first rear plate 271 and the second rear plate 272. According to an embodiment, for example, the second rear plate 272 may be selectively included in the rear plate 270 and may also be omitted therefrom. According to an embodiment, the wearable electronic device 200 or the rear plate 270 may further include a molding member 278 disposed on the inner surface of the second rear plate 272, in the inside thereof. For example, the molding member 278 may be molded of a transparent or a translucent synthetic resin, and the molding member 278 may be disposed to be in contact with the inner surface of the second rear plate 272 while the second rear plate 272 is molded by insert injection. In an embodiment, the second rear plate 272 and the molding member 278 may be manufactured in separate processes, and may be coupled to each other through assembly or attachment processes.

According to an embodiment, the coil assembly 261 may be at least partially embedded in the molding member 278, and may be configured to generate an induced current in response to an external electromagnetic field. In an embodiment, the coil assembly 261 may be electrically connected to the printed circuit board 250 or the auxiliary circuit board 281, and the wearable electronic device 200 may use the induced current generated by the coil assembly 261 as a power source, or may charge the battery 240 by using same. In an embodiment, the molding member 278 may be molded in a state where the coil assembly 261 is disposed in a mold for molding the molding member 278. For example, at the same time that the molding member 278 is molded, the coil assembly 261 may be coupled or fixed to the molding member 278 in a state of being at least partially surrounded by the molding member 278.

According to an embodiment, the auxiliary circuit board 281 may be disposed in a state of facing a first area A1 (e.g., the first rear plate 271) of the rear plate 270. For example, the auxiliary circuit board 281 may be disposed in the seat opening 272a and thus be surrounded by the second rear plate 272 or the molding member 278. In an embodiment, the second rear plate 272 may provide a curved-surface area A2 positioned around the first area A1, and the coil assembly 261 may be disposed in the curved-surface area A2 provided by the second rear plate 272, in the periphery of an area (e.g., the seat opening 272a or the first area A1) in which the auxiliary circuit board 281 is disposed.

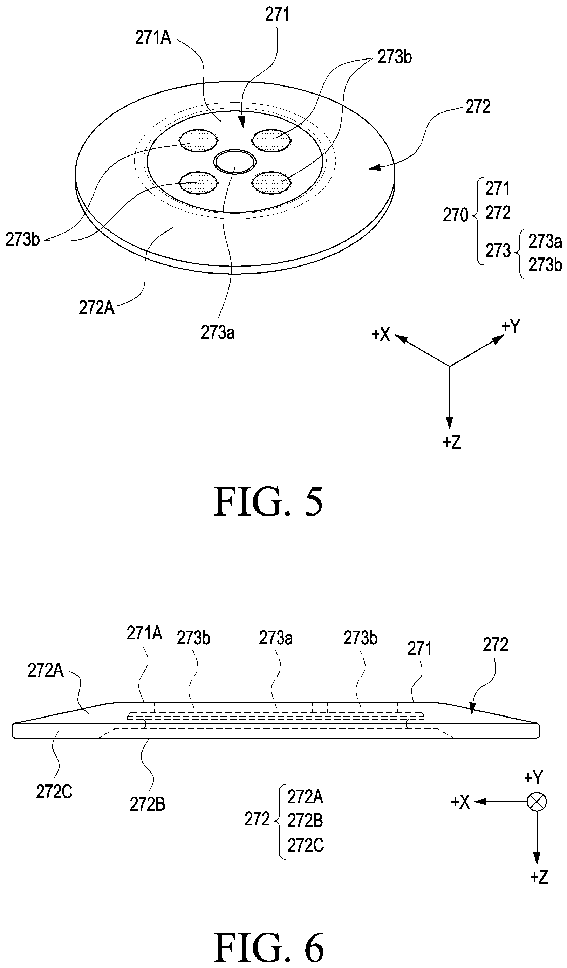

According to an embodiment, in case a portion (e.g., the rear plate 270) of the wearable electronic device 200 is provided as a curved-surface area A2, the coil assembly 261 separated from the auxiliary circuit board 281 may be disposed in a space surrounded by the curved-surface area A2. For example, the auxiliary circuit board 281 may be disposed closer to the first rear plate 271 than the coil assembly 261. In an embodiment, the auxiliary circuit board 281 may include a sensor in which a light-emitting element or a photoelectric conversion element is combined or a sensor (e.g., the sensor module 211 in FIG. 3) using an electrode pad (not shown), and the wearable electronic device 200 may detect user biometric information by using the sensor. In detecting biometric information, since the auxiliary circuit board 281 is disposed closer to the first rear plate 271, the accuracy of the detected biometric information can be further improved if power consumption is the same. In relation thereto, it will be described in more detail with reference to FIG. 6.

According to an embodiment, a sensor (e.g., the sensor module 211 in FIG. 3) disposed on the auxiliary circuit board 281 may detect user biometric information, for example, such as a photo plethysmo graph (PPG), a sleep section, skin temperature, a heart rate, or an electrocardiogram of a user, and the detected biometric information may be stored in the wearable electronic device 200 (e.g., the memory 130 in FIG. 1) or be transmitted to a medical institution in real time, and thus may be used for health management of a user. In transmitting the detected biometric information, a processor (e.g., the processor 120 in FIG. 1) and/or a communication module (e.g., the communication module 190 in FIG. 1) may use the above-described antenna, for example, a part of the housing 210 or the support member 230, and the antenna structure disposed between the display 220 and the support member 230, and/or the coil assembly 261, as an antenna.

Although not illustrated, the sealing member may be positioned between the housing 210 and the rear plate 270. The sealing member may be configured to block moisture and foreign materials from being introduced into the space surrounded by the housing 210 and the rear plate 270 from the outside. In an embodiment, the sealing member may include a double-sided tape disposed between the housing 210 and the front plate 201, and/or an O-ring made of a rubber material, which is provided between the housing 210 and the rear plate 270.

According to an embodiment, the wearable electronic device 200 may include a wireless charging circuit (e.g., a wireless charging circuit provided as a part of the processor 120 in FIG. 1 and/or the power management module 188 in FIG. 1) and the coil assembly 261. In an embodiment, the coil assembly 261 may generate an induced current in response to an external electromagnetic field, and the wireless charging circuit may supply power to the wearable electronic device 200 or may charge the battery 240, by using the induced current generated by the coil assembly 261.

In relation to the following embodiments, the above-described electronic device 101 or 200 in FIG. 1 to FIG. 4 may be referred to, and in relation to configurations which can be easily understood through the above-described embodiments, the reference numerals in drawings may be identically assigned or be omitted, and detailed descriptions thereof may also be omitted.

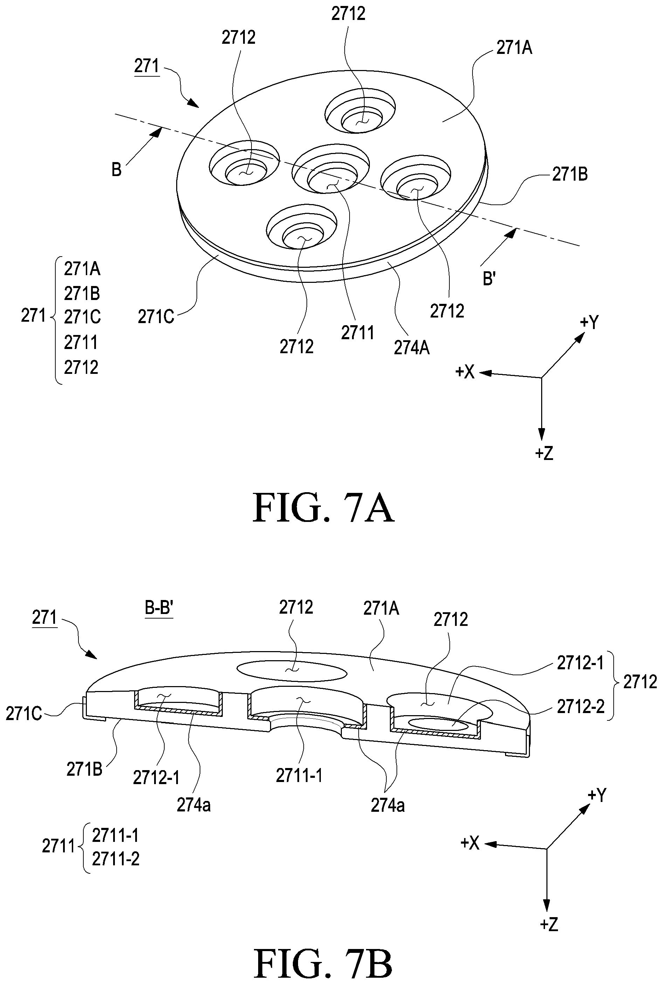

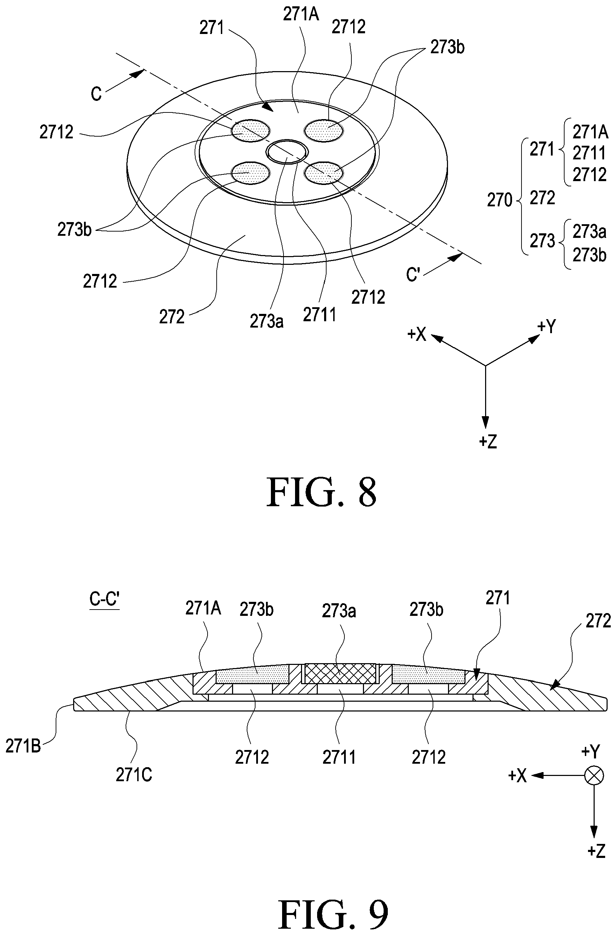

FIG. 5 is a perspective view of a rear plate of a wearable electronic device according to an embodiment of the disclosure. FIG. 6 is a side view of a rear plate of a wearable electronic device according to an embodiment of the disclosure. FIG. 7A is a perspective view of a second rear plate of a wearable electronic device according to an embodiment of the disclosure. FIG. 7B is a cross-sectional view taken along line B-B' in FIG. 7A. FIG. 8 is a perspective view of a rear plate of a wearable electronic device according to an embodiment of the disclosure. FIG. 9 is a cross-sectional view taken along line C-C' in FIG. 8.

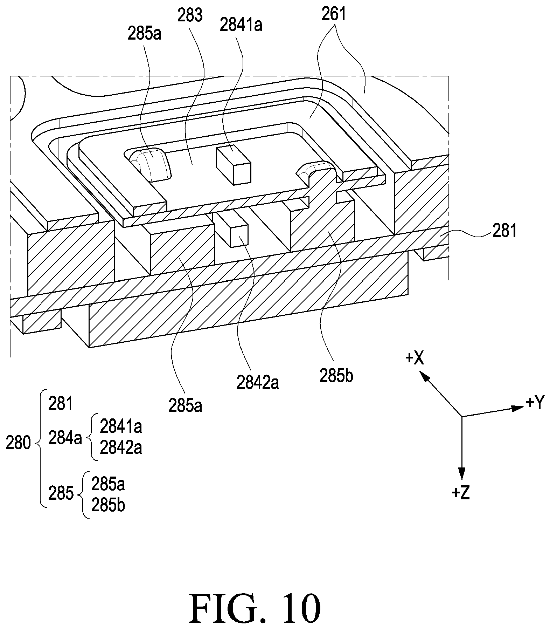

Referring to FIG. 5 to FIG. 9, a wearable electronic device (e.g., the electronic device 101 in FIG. 1 and/or the wearable electronic device 200 in FIG. 2 to FIG. 4) includes a housing (e.g., the housing 210 in FIG. 2 to FIG. 4), a display (e.g., the display 220 in FIG. 2 to FIG. 4), a rear plate 270 (e.g., the rear plate 207 in FIG. 3 and/or the rear plate 270 in FIG. 4), and may include a coil assembly 261 (e.g., the coil assembly 261 in FIG. 4). According to an embodiment, the rear plate 270 may define or form at least a part of the rear surface (e.g., the surface in the -Z-axis direction) of the wearable electronic device 200. According to an embodiment, as described later with reference to FIG. 10 to FIG. 13, a sensor module 280 (e.g., the sensor module 211 in FIG. 3) may be disposed inside the rear plate 270. The sensor module 280 may be disposed closer to the rear plate 270, compared to the display 220. According to an embodiment, the wearable electronic device 200 may be configured to measure biometric information of a user by using the rear plate 270 and/or the sensor module 280.

In an embodiment, the rear plate 270 may constitute at least a part of the rear surface of the wearable electronic device 200. For example, the rear plate 270 may be disposed to face and/or be in contact with the body (e.g., skin) of a user wearing the wearable electronic device 200. For example, the rear plate 270 may be disposed and/or coupled to a housing (e.g., the housing 210 in FIG. 2 and FIG. 3) or the lower end (e.g., the end in -Z-axis direction) of a side bezel structure.

The rear plate 270 includes a first rear plate 271, a second rear plate 272 for surrounding at least a part of the first rear plate 271, and multiple cover members 273 arranged in the first rear plate 271.

In an embodiment, the first rear plate 271 may include a (1-1)th surface 271A facing the outside (e.g., the -Z-axis direction) of the wearable electronic device 200, a (1-2)th surface 271B oriented in a direction opposite to the (1-1)th surface 271A, and a first side surface 271C for connecting the (1-1)th surface 271A and the (1-2)th surface 271B. The first rear plate 271 includes multiple insert areas 2711 and 2712 in which the cover members 273 are arranged. According to an embodiment, the multiple insert areas 2711 and 2712 may be formed through the portion from the (1-1)th surface 271A to the (1-2)th surface 271B, in the first rear plate 271. According to an embodiment, at least a part of the first side surface 271C may face a part of the second rear plate 272.

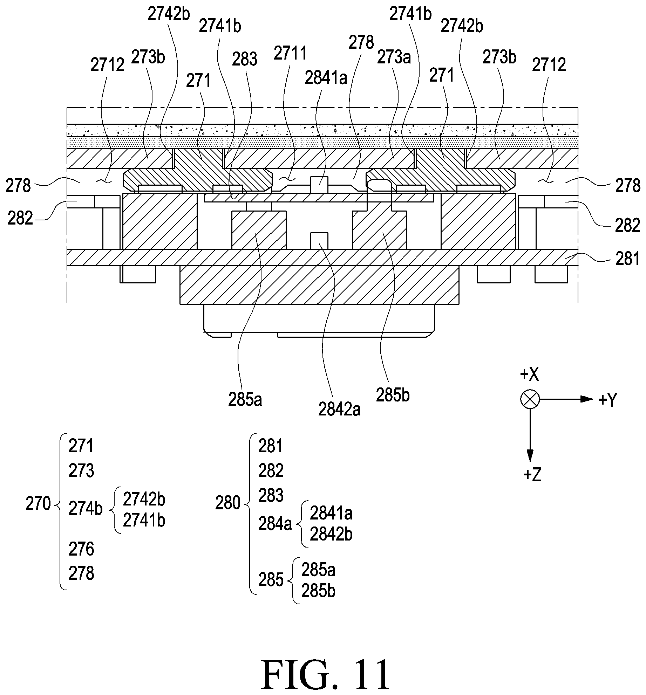

In an embodiment, at least a part of the (1-1)th surface 271A, the (1-2)th surface 271B, and/or the first side surface 271C of first rear plate 271 may be formed or processed to have an optical property which allows light of a specific wavelength to be selectively transmitted and/or blocked. According to an embodiment, at least a part (e.g., the (1-1)th surface 271A) of the first rear plate 271 may include a transparent area for transmitting light outside the wearable electronic device 200 to an optical sensor (or an optical element) (e.g., the optical sensor 282 in FIG. 11) disposed thereinside. For example, the transparent area of the first rear plate 271 may be formed to have high transmittance with respect to light having a wavelength ranging from about 200 nm to about 30 µm or about 300 nm to about 20 µm. For example, the first rear plate 271 may be made by including an insulating material such as coated or colored glass, ceramic, synthetic resin, or sapphire, or by a combination of two or more of the materials. In an embodiment, a light-blocking coating layer may be partially or entirely formed on the first side surface 271C of the first rear plate 271 and/or a second side surface 272C of the second rear plate 272, which faces the first side surface 271C. The light-blocking coating layer may reduce light refraction and cross-talk in a physical space such as the connection portion of the first rear plate 271 and the second rear plate 272 and in an area in which a material is changed.

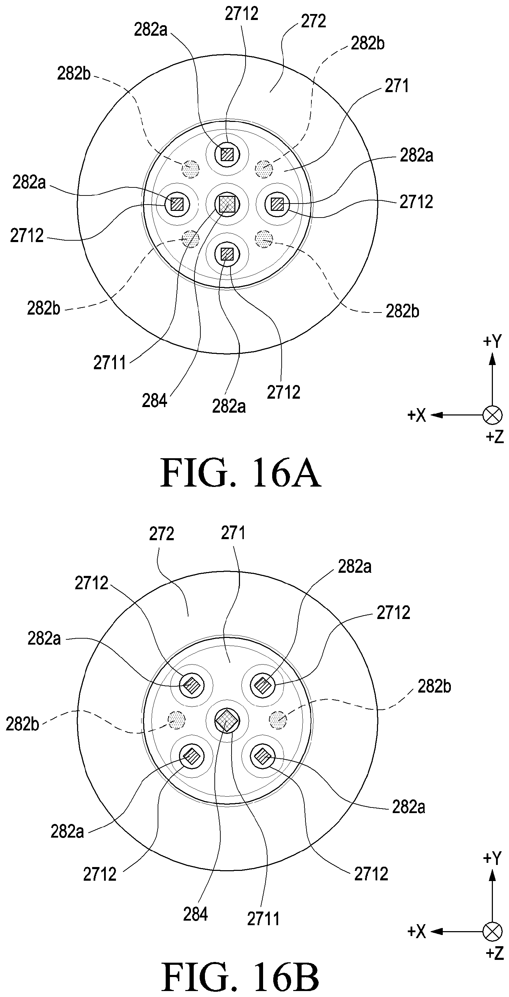

In an embodiment, as will be described later with reference to FIG. 10 to FIG. 14, an optical sensor 282 (e.g., a light-emitting element and/or a photoelectric conversion element) and/or a temperature sensor 284 of the sensor module 280 may be accommodated or disposed in the insert areas 2711 and 2712 of the first rear plate 271. According to an embodiment (e.g., see FIG. 16A to FIG. 17), a part of the optical sensors 282 (e.g., a light-emitting element and/or a photoelectric conversion element) may also be disposed in the insert areas 2711 and 2712, and another part thereof may be disposed around or between the insert areas 2711 and 2712. For example, when seen toward the first rear plate 271, an area of the first rear plate 271, which does not overlap the insert areas 2711 and 2712, may overlap the optical sensor 282 of the sensor module 280.

Referring to FIG. 5 and FIG. 6, in an embodiment, the (1-1)th surface 271A of the first rear plate 271 may be a substantially flat surface. For example, in case the (1-1) surface 271A is a flat surface, compared to the case where the (1-1)th surface 271A is a curved surface, the occurrence of errors or steps may be reduced in case other members (e.g., the second rear plate 272 and/or the cover member 273) are coupled thereto.

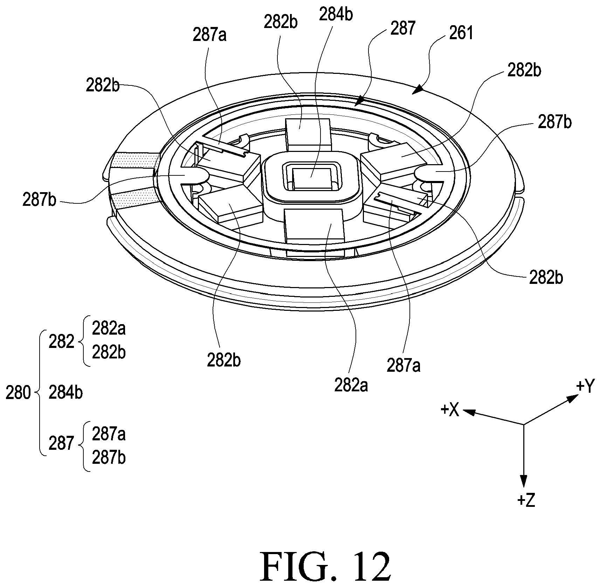

Referring to FIG. 8 and FIG. 9, in an embodiment, the (1-1)th surface 271A may be a substantially curved surface. In case the (1-1) surface 271A is formed as a curved surface as a whole or at least a part including the central portion thereof is formed as a curved surface, compared to the case of a flat surface, the first rear plate 271 may approach closer to or come into closer contact with the body (e.g., skin) of a user wearing the wearable electronic device 200. In case at least a part including the central portion of the first rear plate 271 is formed as a curved surface, it may prevent that external light acting, as noise, on optical-type sensors (e.g., a non-contact-type (or an optical-type) temperature sensor 284b in FIG. 12 and/or a photoelectric conversion element 282a in FIG. 12) arranged inside the first rear plate 271 is introduced thereinto, and thus the measurement accuracy thereof may be further improved. In this case, a shaking phenomenon of the wearable electronic device 200 may be reduced, and the distance between the sensor module 280 and a measurement portion may be reduced so that the thermal conductivity thereof is increased. However, the shape of the (1-1)th surface 271A is not limited, and the (1-1)th surface may at least partially include a flat surface and/or a curved surface.

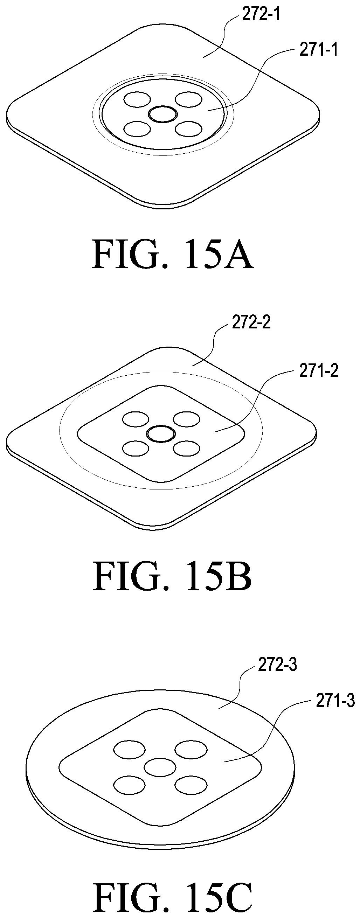

In FIG. 5 to FIG. 9, the first rear plate 271 is illustrated as a circular plate member, but is not limited thereto. In an embodiment (see FIG. 15A to FIG. 15C), the first rear plate 271 may be a polygonal (e.g., quadrilateral) plate member, and for example, may have a corner including a curved surface.

In an embodiment, the insert areas 2711 and 2712 may be holes formed in the first rear plate 271, and may be formed through the portion from the (1-1)th surface 271A to the (1-2)th surface 271B. In an embodiment, the insert areas 2711 and 2712 may include first portions 2711-1 and 2712-1 in which the cover members 273 are seated, and second portions 2711-2 and 2712-2 in which elements of the sensor module 280 are arranged. The first portions 2711-1 and 2712-1 may have shapes recessed by a predetermined height or thickness (e.g., the length in the Z-axis direction) from an opening formed in the (1-1)th surface 271A of the first rear plate 271. The shapes of the first portions 2711-1 and 2712-1 may respond or correspond to the shapes of the cover members 273a and 273b. For example, the height or thickness (e.g., the length in the Z-axis direction) of the first portions 2711-1 and 2712-1 may be greater than or equal to the height or thickness (e.g., the length in the Z-axis direction) of the cover members 273 seated in the first portion 2711-1 and 2712-1. The second portions 2711-2 and 2712-2 may be connected to openings formed in the (1-2)th surface 271B of the first rear plate 271 from ends of the first portions 2711-1 and 2712-1. According to an embodiment, the first portions 2711-1 and 2712-1 of the insert areas 2711 and 2712 may have a maximum width (e.g., a maximum length or diameter on the XY plane) greater than those of the second portions 2711-2 and 2712-2. For example, an opening formed in the first side surface 271C of the first rear plate 271 may have a maximum width (e.g., a maximum length or diameter on the XY plane) or a maximum area, which is greater than an opening formed in the second side surface 272C.

In an embodiment, multiple insert areas 2711 and 2712 may be provided. In an embodiment, the insert areas 2711 and 2712 may include a first insert area 2711 and a second insert area 2712 which are positioned in different areas of the first rear plate 271. According to an embodiment, the first insert area 2711 may be positioned at a central part of the first rear plate 271. According to an embodiment, the first insert area 2711 may be formed as a single hole, and the second insert area 2712 may be formed as multiple (e.g., four) holes. The multiple second insert areas 2712 may be arranged around the single first insert area 2711 to be spaced apart therefrom, and for example, may be spaced apart from the first insert area 2711 at a predetermined interval. For example, an interval between two adjacent second insert areas 2712 may be constant. For example, the multiple second insert areas 2712 may be substantially symmetrical with respect to the first insert area 2711.

However, the shape, the number, and/or the arrangement of the insert areas 2711 and 2712 are not limited to the above-described embodiment. In the illustrated example, the insert areas 2711 and 2712 are illustrated as a circular hollow in a cross section (e.g., a cross section on the XY plane), but are not limited thereto, and the cross section may have various shapes including a polygonal shape and an elliptical shape. For example, at least a part of the side walls of the insert areas 2711 and 2712 may have an angle (e.g., an angle ranging from 0 to about 90 degrees) with respect to the (1-1)th surface 271A of the first rear plate 271. For example, the insert areas 2711 and 2712 may have a tapered shape, and may be a shape having a narrowing width in the direction (e.g., the +Z-axis direction) in which the display 220 is disposed.

The first rear plate 271 further includes a coating area 274 formed on at least one of the side surfaces of the insert areas 2711 and 2712 or the side surface of the cover member 273. Referring to FIG. 7B, a first coating area 274a is formed on at least a part of the side surfaces (or the surfaces) of the insert areas 2711 and 2712. In an embodiment, a second coating area (e.g., the second coating area 274b in FIG. 11) may be formed on at least a part of the side surfaces of the cover members 273. The coating areas 274a and 274b include a light-blocking coating layer and/or a conductive (or thermal conductive) coating layer, and may be configured as a single coating layer or multiple coating layers.

In an embodiment, a (2-1)th coating area (e.g., the (2-1)th coating area 2741b in FIG. 11) may be formed on the side surface of the cover member 273 (e.g., the first cover member 273a) having an inside in which a contact-type temperature sensor (e.g., the contact-type temperature sensor 284a in FIG. 11) (e.g., a thermistor) is disposed. According to an embodiment, the (2-1)th coating area 2741b may include a thermal conductive coating layer (e.g., a metal coating layer). For example, the thermal conductive coating layer (e.g., a metal coating layer) can further improve the conductivity of external heat transferred to the contact-type temperature sensor 284a, and further improve the accuracy of temperature measurement. For example, the (2-1)th coating area 2741b may also further include a light-blocking coating layer on which a thermal conductive coating layer is laminated or which is laminated on the thermal conductive coating layer.

In an embodiment, a (2-2)th coating area (e.g., the (2-2)th coating area 2742b in FIG. 11) may include a light-blocking coating layer. According to an embodiment, the (2-2)th coating area 2742b may be formed on the side surface of the cover member 273 (e.g., the second cover member 273b) in which the optical sensor 282 is disposed. According to an embodiment, although not illustrated in drawings, the (2-2)th coating area 2742b may be formed on the side surface of the cover member 273 (e.g., the first cover member 273a) having an inside in which a non-contact-type (or an optical-type) temperature sensor (e.g., the non-contact-type temperature sensor 284b in FIG. 12) is disposed.

According to an embodiment, light-blocking coating layers of the first coating area 274a and the (2-2)th coating area (e.g., the (2-2)th coating area 2742b in FIG. 11) may prevent the generation of cross-talk in the optical sensor 282 (e.g., a light-emitting element and a photoelectric conversion element) and/or the non-contact-type temperature sensor 284 (e.g., the non-contact-type temperature sensor 284b in FIG. 12) which are arranged inside the cover members 273. For example, in case the first coating area 274a and/or the (2-2)th coating area (e.g., the (2-2)th coating area 2742b in FIG. 11) is formed, compared to the case of not being formed, light refraction and cross-talk can be reduced in a physical space such as the connection portion of the insert areas 2711 and 2712 and the cover member 273 and in an area in which a material is changed.

According to an embodiment, the light-blocking coating layers of the first coating area 274a and/or the (2-2)th coating area (e.g., the (2-2)th coating area 2742b in FIG. 11) may be formed by coating oxide, nitride, black ink, or by a combination of the above materials. For example, oxide or nitride can be coated on the surface in the form of a thin layer through a process such as deposition. For example, the deposition process may include sputtering, chemical vapor deposition (CVD), and thermal deposition (thermal deposition) methods. According to an embodiment, the light-blocking coating layers may also be formed by laminating (e.g., printing or spraying) a light-blocking material layer such as black ink on an oxide layer and/or a nitride layer.

In an embodiment, the second rear plate 272 may include a (2-1)th surface 272A facing the outside (e.g., the -Z-axis direction) of the wearable electronic device 200, a (2-2)th surface 272B oriented in a direction opposite to the (2-1)th surface 272A, and a second side surface 272C for connecting a portion between the (2-1)th surface 272A and the (2-2)th surface 272B. According to an embodiment, the (2-1)th surface 272A may at least partially include a curved surface. In an embodiment, the first rear plate 271 may include a seat opening (e.g., the seat opening 272a in FIG. 4) formed at the central part thereof. In an embodiment, when seen from above the rear plate (270) (or in the Z-axis direction), the second rear plate 272 may have a loop shape forming or defining the seat opening, and the first rear plate 271 may be disposed in the seat opening (e.g., the seat opening 272a in FIG. 4) of the second rear plate 272. For example, the shape of the seat opening 272a and the shape of the first rear plate 271 may correspond to each other.

In the illustrated embodiment, the second rear plate 272 is illustrated in a substantially circular loop shape when seen in the Z-axis direction, but is not limited thereto, and the second rear plate 272 may have a polygonal loop shape. For example, the outer edge of the second rear plate 272 may have a circular shape and the seat opening 272a may have a polygonal shape, and the vice versa may also be possible. In an embodiment, the first rear plate 271 may be coupled to the second rear plate 272 by a sealing member or an adhesive member or such as double-sided tape. For example, a sealing structure and/or a waterproof structure may be provided between the first rear plate 271 and the second rear plate 272, in the seat opening 272a.

In an embodiment, the second rear plate 272 may be formed to be substantially opaque to block light outside the wearable electronic device 200. According to an embodiment, the second rear plate 272 may block external light acting as noise on the optical sensor 282 disposed thereinside or the optical sensor 282 disposed inside the first rear plate 271 from being introduced thereinto. For example, the second rear plate 272 may include an opaque area in at least a part thereof. For example, at least a part of the second rear plate 272, which includes the (2-1)th surface 272A, may also be coated or colored in a color (e.g., black). For example, the second rear plate 272 may be made by including an insulating material such as coated or colored glass, ceramic, synthetic resin, or sapphire, or by a combination of at least two of the materials.

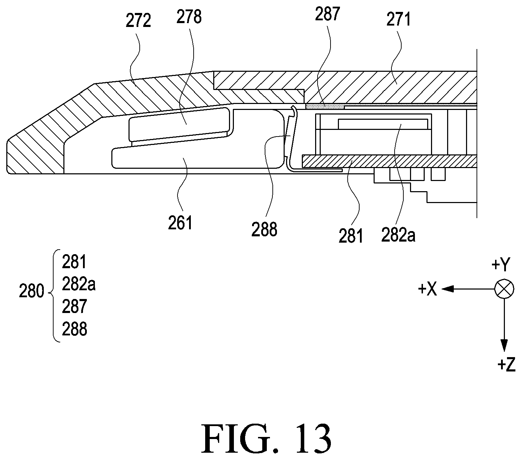

In an embodiment, an electrode structure (not shown) for electrochemically measuring biometric information of a user may be disposed on a surface (e.g., the inner surface or the surface in the +Z-axis direction) of the rear plate 270. According to an embodiment, it may also be understood that the electrode structure (not shown) disposed on the rear plate 270 is included in the sensor module 280. In an embodiment, the electrode structure (not shown) disposed on the rear plate 270 may be electrically connected to a printed circuit board (e.g., the printed circuit board 250 in FIG. 4) through an electrical member (e.g., the bridge connector 287 in FIG. 12 and FIG. 13). In an embodiment, as described later, in case the sensor module 280 includes the second auxiliary circuit board 283 on which the temperature sensor 284 is disposed, the electrode structure (not shown) disposed on the rear plate 270 may be electrically connected to the printed circuit board through the second auxiliary circuit board 283.

In an embodiment, multiple cover member 273 may be provided, and may be arranged in the insert areas 2711 and 2712 (e.g., the first portions 2711-1 and 2712-1) of the first rear plate 271, respectively. The cover members 273 may form a space for accommodating a sensor or an element of the sensor module 280 together with the insert areas 2711 and 2712 in a state of being seated in the first portion 2711-1 or 2712-1. For example, the cover members 273 may be made by including an insulating material such as coated or colored glass, ceramic, synthetic resin, sapphire, silicon, zinc sulfide, and germanium, or by a combination of at least two of the materials.

In an embodiment, the cover members 273 may include the first cover member 273a disposed in the first insert area 2711 and the second cover member 273b disposed in the second insert area 2712. According to an embodiment, the temperature sensor 284 may be disposed in the first insert area 2711 or between the first insert area 2711 and the first cover member 273a, and the optical sensor 282 may be disposed in the second insert area 2712 or between the second insert area 2712 and the second cover member 273b. According to an embodiment, the optical sensor 282 may be disposed in the first insert area 2711 or between the first insert area 2711 and the first cover member 273a, and the temperature sensor 284 may be disposed in the second insert area 2712 or between the second insert area 2712 and the second cover member 273b.

According to an embodiment, the temperature sensor 284 may include a contact-type temperature sensor (e.g., the contact-type temperature sensor 284a in FIG. 10 and FIG. 11) such as a thermistor, and/or a non-contact-type (or an optical-type) temperature sensor (e.g., the non-contact-type temperature sensor 284b in FIG. 12) such as an infrared temperature sensor or an infrared (IR) thermopile. According to an embodiment, the optical sensor 282 may include a light-emitting element (e.g., an LED) and/or a photoelectric conversion element (or a light-receiving element) (e.g., a photodiode or a phototransistor)).

In an embodiment, the cover members 273 may be configured to transmit and/or block light of a specific wavelength, generated from the optical sensor 282 (e.g., a light-emitting element or a photoelectric conversion element) or a non-contact-type temperature sensor (e.g., the non-contact-type temperature sensor 284b in FIG. 12) thereinside. For example, the cover members 273 may be formed by coating a material having permeability with respect to light of a specific wavelength on a substantially transparent material. According to an embodiment, the cover member 273 (e.g., the second cover member 273b) having the optical sensor 282 (e.g., a light-emitting element or a photoelectric conversion element) disposed thereinside may be formed to transmit light having a wavelength in a first designated range (e.g., a range of about 300 nm to about 1.5 µm). In an embodiment, in case a non-contact-type temperature sensor (e.g., the non-contact-type temperature sensor 284b in FIG. 12) is disposed inside the cover member 273 (e.g., the first cover member 273a), the cover member 273 may be formed to transmit light having a wavelength in a second designated range (e.g., a range of about 1 to about 20 µm).

According to an embodiment, the cover members 273 may be seated in the first portions 2711-1 and 2712-1 of the insert areas 2711 and 2712. For example, the cover members 273 may be coupled to the first portions 2711-1 and 2712-1 by a sealing member or an adhesive member such as a double-sided tape. For example, in case the cover members 273 are coupled to the first rear plate 271, the outer surfaces (e.g., the surface in the -Z-axis direction) of the cover members 273 may form a continuous surface with the (1-1)th surface 271A of the first rear plate 271. In an embodiment, in case the (1-1)th surface 271A of the first rear plate 271 is a curved surface, the outer surfaces of the cover members 273, which are adjacent thereto, may be formed to have a curved surface, and for example, may be formed to have a curved surface having a curvature substantially the same as the curvature of the curved surface of the (1-1) surface 271A so that the cover members 273 forms a smooth curved surface with the first rear plate 271. In an embodiment, in case the (1-1)th surface 271A of the first rear plate 271 is a substantially flat surface, the outer surfaces of the cover members 273 may be a flat surface. The shape of the cover members 273 may correspond to the shape of the insert areas 2711 and 2712 (e.g., the first portions 2711-1 and 2712-1). In the illustrated embodiment, the cover member 273 is illustrated as a circular plate member, but is merely an example and is not limited thereto, and the cover members 273 may have various shapes including an elliptical shape or a polygonal shape.

In an embodiment, the cover members 273 may also have a different shape and/or different curvature from that of the first rear plate 271. As an example, in a case in which the first rear plate 271 is flat, the cover members 273 can be curved and can be cofigured to protrude outwardly from a plane of the first rear plate 271.

Hereinafter, referring to FIG. 10 to FIG. 14, elements including the sensor module 280 disposed inside the rear plate 270 according to an embodiment described with reference to FIG. 5 to FIG. 9 will be described in more detail.

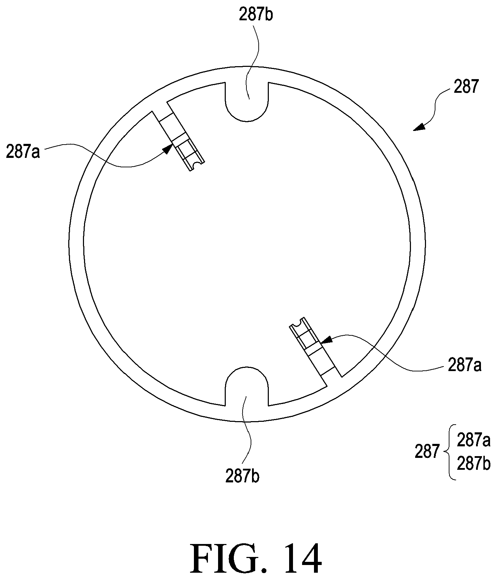

FIG. 10 is a cross-sectional perspective view of a wearable electronic device according to an embodiment of the disclosure. FIG. 11 is a cross-sectional view of a wearable electronic device according to an embodiment of the disclosure. FIG. 12 is a perspective view of a state in which a second rear plate of a wearable electronic device is removed according to an embodiment of the disclosure. FIG. 13 is a partial cross-sectional view of a wearable electronic device according to an embodiment of the disclosure. FIG. 14 is a plan view of a bridge connector of a wearable electronic device according to an embodiment of the disclosure.

Referring to FIG. 10 to FIG. 14, the sensor module 280 includes an optical sensor 282, a first auxiliary circuit board 281, a temperature sensor 284, a second auxiliary circuit board 283, and/or a bridge connector 287. The first rear plate 271 in FIG. 11 and FIG. 14 may refer to the first rear plate 271 in FIG. 4 to FIG. 9. The second rear plate 272 in FIG. 14 may refer to the second rear plate 272 in FIG. 4 to FIG. 6, FIG. 8, and FIG. 9.

In an embodiment, the optical sensor 282 may include a light-emitting element (e.g., an LED) and/or a photoelectric conversion element (or a light-receiving element). For example, the photoelectric conversion element may be a photodiode or a phototransistor.

The optical sensor 282 of the sensor module 280 is disposed on a surface (e.g., the surface in the -Z-axis direction) of the first auxiliary circuit board 281, which faces the rear plate 270. According to an embodiment, the optical sensor 282 may be disposed in an area corresponding to the second insert area 2712 of the first rear plate 271 on the first auxiliary circuit board 281. The optical sensor 282 may be accommodated in a space of the first rear plate 271, which is surrounded by the second cover member 273b and the second insert area 2712.

According to an embodiment, the sensor module 280 may include multiple light-emitting elements and a single photoelectric conversion element arranged in the second insert area 2712. However, the number and arrangement of photoelectric conversion elements are not limited to the above-described embodiment, and for example, the sensor module 280 may include multiple photoelectric conversion elements and may include a single light-emitting element, also. According to the embodiment, the light-emitting element and/or the photoelectric conversion element may also be arranged in the first insert area 271. According to embodiments, the light-emitting element and/or the photoelectric conversion element may also be disposed in a space between the second insert areas 2712 adjacent to each other.

In an embodiment, in a state where the optical sensor 282 and the second cover member 273b are arranged in the second insert area 2712, the remaining spaces of the second insert area 2712 may be filled with a filling member 278. For example, the filling member 278 may include a thermal interface material (TIM).

According to an embodiment, in the wearable electronic device 200, the light-emitting element and/or the photoelectric conversion element may be used as a sensor (e.g., the first sensor module 211 in FIG. 3) for detecting biometric information (e.g., a heart rate (an HRM or an HRV)) of a user, through a selective combination of the light-emitting element and/or the photoelectric conversion element. For example, the optical sensor 282 may collect biometric information, for example, raw data for measuring a blood pressure, blood flow, heart rate (an HRM or an HRV), body temperature, respiratory rate, oxygen saturation (SpO₂), cardiopulmonary sound detection, blood sugar, waist circumference, height, weight, body fat, calorie consumption, brain wave, voice, skin resistance, electromyogram, and/or electrocardiopram of a user. For example, the wearable electronic device 200 may generate biometric information by collecting and processing the raw data. As an example, the wearable electronic device 200 may collect pulse wave data by using a heart rate variability (HRV) sensor and/or a heart rate monitor (HRM) sensor through a combination of the light-emitting element and/or the photoelectric conversion element. For example, the wearable electronic device 200 may process the collected pulse wave data to provide biometric information such as an average heart rate, heart rate distribution, and/or blood vessel aging.

In an embodiment, in a state where the wearable electronic device 200 is worn on the body of a user, at least a part of the rear plate 270 may come into contact with the body of the user, and in case there is a request or order for detecting biometric information, a processor (e.g., the processor 120 in FIG. 1) may be configured to emit light by using a light-emitting element and to detect light reflected from the body of the user by using the photoelectric conversion element. The "reflected light" may mean light reflected from the body of the user in an area in which the body is substantially in contact with the rear plate 270. The wearable electronic device 200 and/or a processor (e.g., the processor 120 in FIG. 1) may detect or determine biometric information of a user based on the reflected light and/or the detected light. According to an embodiment, the rear plate 270 may be configured to at least partially transmit the light emitted from the light-emitting element disposed in the sensor module 280 toward the outside of the wearable electronic device 200 and/or the light received by the light-receiving element from the outside of the electronic device 200.

The sensor module 280 includes the second auxiliary circuit board 283 on which the temperature sensor 284 is disposed. For example, the second auxiliary circuit board 283 may be a flexible printed circuit board. The second auxiliary circuit board 283 is disposed between the rear plate 270 (e.g., the first rear plate 271) and the first auxiliary circuit board 281. For example, the second auxiliary circuit board 283 may be coupled to a second surface (e.g., the surface in the +Z-axis direction) of the first rear plate 271 by an adhesive member (e.g., a double-sided tape) or an adhesive material. For example, the second auxiliary circuit board 283 may be disposed to overlap the insert areas 2711 and 2712 in which the temperature sensor 284 is disposed with reference to the thickness direction (e.g., the Z-axis direction) of the wearable electronic device 200. According to an embodiment, in case the temperature sensor 284 is disposed in the first insert area 2711, the second auxiliary circuit board 283 may be disposed to face the first cover member 273a, and may close an opening formed at one end (e.g., the end in the +Z-axis direction) of the first insert area 2711 or formed in the second surface (e.g., the surface in the +Z-axis direction) of the first rear plate 271. According to an embodiment, in case the temperature sensor 284 is disposed in the second insert area 2712, the second auxiliary circuit board 283 may be disposed to face the second cover member 273b, and may close an opening formed at one end (e.g., the end in the +Z-axis direction) of the second insert area 2712 or formed in the second surface (e.g., the surface in the +Z-axis direction) of the first rear plate 271.

According to an embodiment, the second auxiliary circuit board 283 may be electrically connected to the first auxiliary circuit board 281 through a conductive connection member 285a or 285b (e.g., a C-clip) such as a pin member. For example, the connection member 285a and 285b may be provided as multiple (e.g., two) connection members 285a and 285b arranged while having the temperature sensor 284 interposed therebetween. According to an embodiment, for example, the second auxiliary circuit board 283 may be electrically connected to a printed circuit board (e.g., the printed circuit board 250 in FIG. 4) through the first auxiliary circuit board 281. For example, temperature data (e.g., data about a body temperature of a user) measured by the temperature sensor 284 may be provided to a processor (e.g., the processor 120 in FIG. 1) disposed on the first auxiliary circuit board 281 or the printed circuit board 250.

According to an embodiment, the temperature sensor 284 may be disposed on a surface (e.g., the surface in the -Z-axis direction) of the second auxiliary circuit board 283, which faces the rear plate 270. According to an embodiment, the temperature sensor 284 may be disposed in an area corresponding to the first insert area 2711 of the first rear plate 271 on the second auxiliary circuit board 283. The temperature sensor 284 may be accommodated in a space of the first rear plate 271, which is surrounded by the first cover member 273a and the first insert area 2711. In an embodiment, in a state where the temperature sensor 284 and the first cover member 273a are arranged in the first insert area 2711, the remaining spaces of the first insert area 2711 may be filled with the filling member 278. For example, the filling member 278 may include a thermal interface material (TIM). In an embodiment, at least a part of the coil assembly 261 may be disposed on a surface (e.g., the surface in -Z-axis direction) of the second auxiliary circuit board 283. For example, the temperature sensor 284 may be disposed at the central part of the second auxiliary circuit board 283, and the charging coil assembly 261 may be disposed around the temperature sensor 284 to be spaced apart therefrom.

Referring to FIG. 10 and FIG. 11, in an embodiment, the temperature sensor 284 of the sensor module 280 may be a contact-type temperature sensor 284a such as a thermistor. In an embodiment, temperature sensors 2841a and 2842a may include multiple (e.g., two) thermistors. The first temperature sensor 2841a is disposed on the second auxiliary circuit board 283, and a second temperature sensor 2842a is disposed at a position corresponding to the first temperature sensor 2841a on the first auxiliary circuit board 281. For example, the first temperature sensor 2841a and the second temperature sensor 2842a may be aligned along one axis (e.g., the Z-axis). For example, the multiple (e.g., two) connection members 285a and 285b (e.g., a C-clip) may be arranged on the first auxiliary circuit board 281 while having the second temperature sensor 2842a interposed therebetween. For example, the wearable electronic device 200 or the sensor module 280 may derive additional biometric information (e.g., a heart temperature) from the temperature of radiant heat conducted from a user based on the temperatures measured by the two temperature sensors 2841a and 2842a. In the above-described embodiment, the temperature sensors 2841a and 2842a are exemplified as thermistors, but the type of the temperature sensor 284 is not limited. For example, the temperature sensor 284 may be a contact-type temperature sensor such as a thermocouple, resistance temperature detector (RTDs), or a bimetal temperature sensor (a bimetal thermometer), in addition to a thermistor. In an embodiment, the temperature sensor 284 may be a non-contact-type temperature sensor (e.g., the non-contact-type temperature sensor 284b in FIG. 12) such as an infrared (IR) temperature sensor (thermopile).

In an embodiment, the wearable electronic device 200 may further include an electrode structure (or an electrode pad) (not shown) and/or a pressure sensor (not shown) disposed on the rear plate 270. According to an embodiment, the electrode structure may be disposed on the inner surface (e.g., the surface in the +Z-axis direction) of the rear plate 270, and for example, may be exposed to the outer surface (e.g., the surface in the -Z-axis direction) of the rear plate 270. The electrode structure and/or the pressure sensor disposed on the rear plate 270 may be electrically connected to the first auxiliary circuit board 281. For example, the electrode structure and/or the pressure sensor may be in direct contact with the body of a user, and thus be used to detect user biometric information. For example, the wearable electronic device 200 may provide biometric information such as an electrocardiogram, an electroencephalogram, an amount of body fat (by a bioelectrical impedance analysis), and galvanic skin response based on the raw data detected by the electrode structure and/or pressure sensor.

Referring to FIG. 12 and FIG. 13, in an embodiment, the wearable electronic device 200 may further include the bridge connector 287 for electrically connecting an electrode structure (not shown) disposed on a plate and the first auxiliary circuit board 281. According to an embodiment, the bridge connector 287 may be disposed between the rear plate 270 (e.g., the first rear plate 271 and/or the second rear plate 272) and the sensor module 280. According to the embodiment, the bridge connector 287 may be disposed so as not to overlap the coil assembly 261 (e.g., the coil assembly 261 in FIG. 4) (e.g., a charging coil) with reference to the thickness direction of the wearable electronic device 200. According to an embodiment, the coil assembly 261 may be disposed outside or around the bridge connector 287 to be spaced apart therefrom.

Referring to FIG. 13, according to an embodiment, the coil assembly 261 may be disposed in a form of surrounding the bridge connector 287. According to an embodiment, the wearable electronic device 200 may further include a wireless charging circuit (e.g., the power management module 188 in FIG. 1 and/or a wireless charging circuit provided as a part of the processor 120 in FIG. 1). According to an embodiment, the coil assembly 261 may generate an induced current in response to an external electromagnetic field, and the wireless charging circuit may supply power to the wearable electronic device 200 or may charge a battery (e.g., the battery 240 in FIG. 4), by using the induced current generated by the coil assembly 261.

According to an embodiment, the wearable electronic device 200 or the rear plate 270 may further include a molding member (e.g., the molding member 278 in FIG. 4) disposed on the inner surface of the second rear plate 272, in the inside thereof. According to an embodiment, the coil assembly 261 may be at least partially embedded in the molding member 278.

In an embodiment, the bridge connector 287 may be fixedly coupled to the inner surface of the rear plate 270 (e.g., the first rear plate 271 and/or the second rear plate 272). For example, the bridge connector 287 may be soldered to the rear plate 270 (e.g., the first rear plate 271 and/or the second rear plate 272) and/or a support member (e.g., the support member 240 in FIG. 4). For example, the bridge connector 287 may be a flexible printed circuit board, and may be made by including a material having high heat resistance, such as a liquid polymer (a liquid crystal polymer). In an embodiment, the bridge connector 287 may include a first protruding part 287a electrically connected to an electrode structure (not shown) disposed on the rear plate (270), and a second protruding part 287b electrically connected to the first auxiliary circuit board 281. According to an embodiment, a pad including a coverlay (or a photosensitive coverlay) for insulating and/or protecting a circuit may be disposed at an open end of the first protruding part 287a. According to an embodiment, the first protruding part 287a and the second protruding part 287b may be spaced apart from each other, and for example, each thereof may be provided in a plural (e.g., two) number. For example, the multiple (e.g., two) first protruding parts 287a may be arranged symmetrically to each other, and the multiple (e.g., two) second protruding parts 287b may be arranged symmetrically to each other.

According to an embodiment, the bridge connector 287 and/or the flexible connection member 288 may be an insert structure according to an embodiment of the disclosure, and may provide a circuit structure for electrically connecting a portion between the first auxiliary circuit board 281 and an electrode structure (not shown) of the rear plate 270, which are physically separated and spaced apart from each other by an insert structure in which sensing elements (e.g., the temperature sensor 284 and/or the optical sensor 282) of the sensor module 280 are arranged in the insert areas 2711 and 2712.

However, the electrical connection structure between the electrode structure (not shown) and the first auxiliary circuit board 281 of the disclosure is not limited to the above-described embodiment, and may also be provided by a contact pad provided between the electrode structure and the first auxiliary circuit board 281. For example, the contact pad may be made by including a conductive polymer such as a conductive silver composite material, conductive epoxy, or conductive silicone. According to an embodiment of the disclosure, since the bridge connector 287 is provided by a fixed coupling method (e.g., soldering), compared to the electrical connection structure using the contact pad, contact instability caused by external factors (e.g., external physical impacts) can be reduced.

FIG. 15A is a perspective view of a rear plate of a wearable electronic device according to an embodiment of the disclosure. FIG. 15B is a perspective view of a rear plate of a wearable electronic device according to an embodiment of the disclosure. FIG. 15C is a perspective view of a rear plate of a wearable electronic device according to an embodiment of the disclosure.

The embodiments of FIG. 15A to FIG. 15C do not technically contradict or conflict with the embodiments of FIG. 5 to FIG. 15C, and be combined with the embodiments of FIG. 5 to FIG. 15C.

First rear plates 271-1, 271-2, and 271-3 and second rear plates 272-1, 272-2, and 272-3 according to the embodiments of FIG.15A to FIG. 15C may have all the same features as the first rear plate 271 and the second rear plate 272 according to the embodiments of FIG. 5 to FIG. 14, except for the shapes thereof. The embodiments of FIG. 15A to FIG. 15C will be described centering on the difference from the embodiments of FIG. 5 to FIG. 14, and overlapping descriptions thereof may be omitted below.

Referring to FIG. 15A, the shape of the first rear plate 271-1 may be a circle, and the shape of the second rear plate 272-1 disposed to surround the edge of the first rear plate 271-1 may be a quadrilateral shape and the quadrilateral shape may be a chamfered shape.

Referring to FIG. 15B, the shape of the first rear plate 271-2 may be a quadrilateral shape, and the quadrilateral shape may be a chamfered shape. The shape of the second rear plate 272-2 disposed to surround the edge of the first rear plate 271-2 may be a quadrilateral shape, and the quadrilateral shape may be a chamfered shape.

Referring to FIG. 15C, the shape of the first rear plate 271-3 may be a quadrilateral shape, and the quadrilateral shape may be a chamfered shape. The shape of the second rear plate 272-3 disposed to surround the edge of the first rear plate 271-3 may be a circle.

In case the first rear plate 271-1, 271-2, or 271-3 has a quadrilateral shape as the first rear plate 271-2 or 271-3 in FIG. 15B and FIG. 15C, compared to the case in which the first rear plate 271-1, 271-2, or 271-3 has a circular shape, the spaced distance between a photoelectric conversion element (e.g., the photoelectric conversion element 282a in FIG. 12) and a light-emitting element (e.g., the light-emitting element 282b in FIG. 12) which are arranged inside the first rear plate 271-1, 271-2, or 271-3, may be increased and become various, and thus it may be more advantageous in measuring biometric information such as a heart rate.

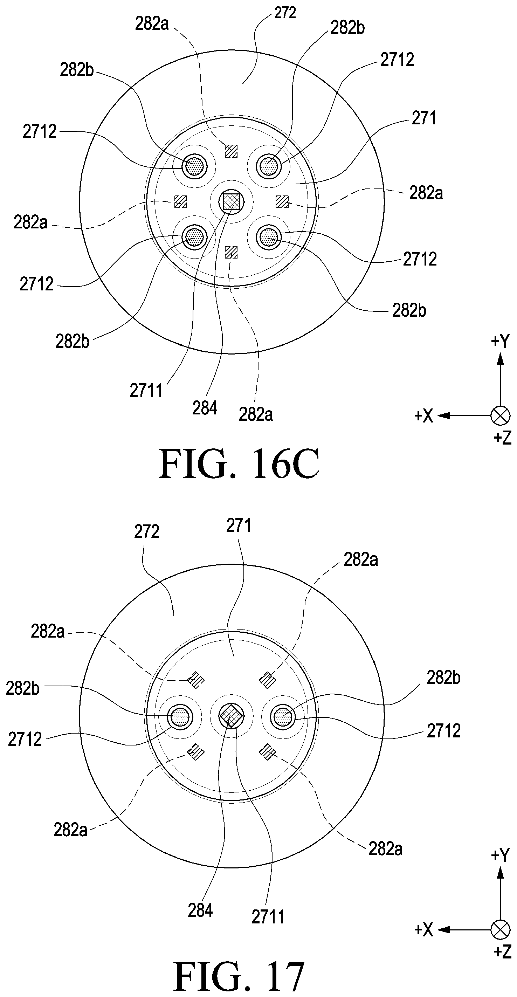

FIG. 16A is a plan view of a rear plate of a wearable electronic device according to an embodiment of the disclosure. FIG. 16B is a plan view of a rear plate of a wearable electronic device according to an embodiment of the disclosure. FIG. 16C is a plan view of a rear plate of a wearable electronic device according to an embodiment of the disclosure. FIG. 17 is a plan view of a rear plate of a wearable electronic device according to an embodiment of the disclosure.

The embodiments of FIG. 16A to FIG. 17 do not technically contradict or conflict with the embodiments of FIG. 5 to FIG. 15C, and be combined with the embodiments of FIG. 5 to FIG. 15C.

The second rear plate 272 of the embodiments of FIG. 16A to FIG. 17 may refer to the second rear plate 272 of the embodiments of FIG. 5 to FIG. 14. The optical sensors 282a and 282b and the temperature sensor 284 of the embodiments of FIG. 16A to FIG. 17 may refer to the optical sensors 282a and 282b and the temperature sensor 284 of the embodiments of FIG. 5 to FIG. 14. All or a part of the configuration of the first rear plate 271 of the embodiments of FIG. 16A to FIG. 17 may be the same as the configuration of the first rear plate 271 of the embodiments of FIG. 5 to FIG. 14. The first rear plate 271 according to the embodiment of FIG. 17 may have all the same configuration and feature as the configuration (e.g., the first coating layer 274a in FIG. 7B) and feature of the first rear plate 271 according to the embodiments of FIG. 5 to FIG. 14, except for the number and arrangement of the insert areas 2711 and 2712. The embodiments of FIG. 16A to FIG. 17will be described centering on the difference from the embodiments of FIG. 5 to FIG. 14, and overlapping descriptions thereof may be omitted below.

Referring to FIG. 16A to FIG. 17, in an embodiment, the first rear plate 271 may include a first insert area 2711 formed at the central part thereof and multiple second insert areas 2712 formed around the first insert area 2711. According to an embodiment, a temperature sensor 284 (e.g., a contact-type or a non-contact-type temperature sensor) may be disposed in the first insert area 2711. For example, the temperature sensor 284 may be a contact-type temperature sensor (e.g., the contact- type temperature sensor 284a in FIG. 10 and FIG. 11) such as a thermocouple, resistance temperature detectors (RTDs), or a bimetal temperature sensor (a bimetal thermometer), in addition to a thermistor. In an embodiment, the temperature sensor 284 may be a non-contact (or an optical-type) temperature sensor (e.g., the non-contact-type temperature sensor 284b in FIG. 12) such as an infrared (IR) temperature sensor (thermopile).

Referring to FIG. 16A, according to an embodiment, photoconversion elements 282a (e.g., photodiodes or a phototransistors) may be arranged in multiple (e.g., four) second insert areas 2712, respectively. According to an embodiment, light-emitting elements 282b (e.g., LEDs) may be arranged around the second insert areas 2712. According to an embodiment, the photoconversion elements 282a may be arranged at portions between the second insert areas 2712 adjacent to each other, respectively.

Referring to FIG. 16B, according to an embodiment, photoconversion elements 282a (e.g., photodiodes or phototransistors) may be arranged in multiple (e.g., four) second insert areas 2712, respectively. According to an embodiment, light-emitting elements 282b (e.g., LEDs) may be arranged around the second insert areas 2712. According to an embodiment, the light-emitting elements 282b may be arranged only in some areas between the second insert areas 2712 adjacent to each other. For example, the light-emitting elements 282b may be provided in a smaller number than the photoconversion elements 282a. For example, multiple (e.g., two) light-emitting elements 282b may be respectively arranged at positions which are between the second insert areas 2712 adjacent to each other and are symmetrical with respect to the temperature sensor 284.

Referring to FIG. 16C, according to an embodiment, light-emitting elements 282b (e.g., LEDs) may be arranged in multiple (e.g., four) second insert areas 2712, respectively. According to an embodiment, photoconversion elements 282a (e.g., photodiodes or phototransistors) may be arranged around the second insert areas 2712.

Referring to FIG. 17, light-emitting elements 282b (e.g., LEDs) may be arranged in multiple (e.g., two) second insert areas 2712, respectively. According to an embodiment, photoconversion elements 282a (e.g., photodiodes or phototransistors) may be arranged around the second insert areas 2712. According to an embodiment, the multiple (e.g., four) photoconversion elements 282a may be arranged at portions between the second insert areas 2712 adjacent to each other, respectively. For example, the multiple (e.g., four) light-emitting elements 282a may be respectively arranged at positions which are between the second insert areas 2712 adjacent to each other and are symmetrical with respect to the temperature sensor 284, each of the multiple light-emitting elements being disposed as one pair.

Referring to FIG. 16A to FIG. 17, according to an embodiment, the temperature sensor 284 and the optical sensors 282a and 282b, which are arranged in the insert areas 2711 and 2712 or around the insert areas 2711 and 2712, may be arranged on a first auxiliary circuit board (e.g., the first auxiliary circuit board 281 in FIG. 11) inside the first rear plate 271.

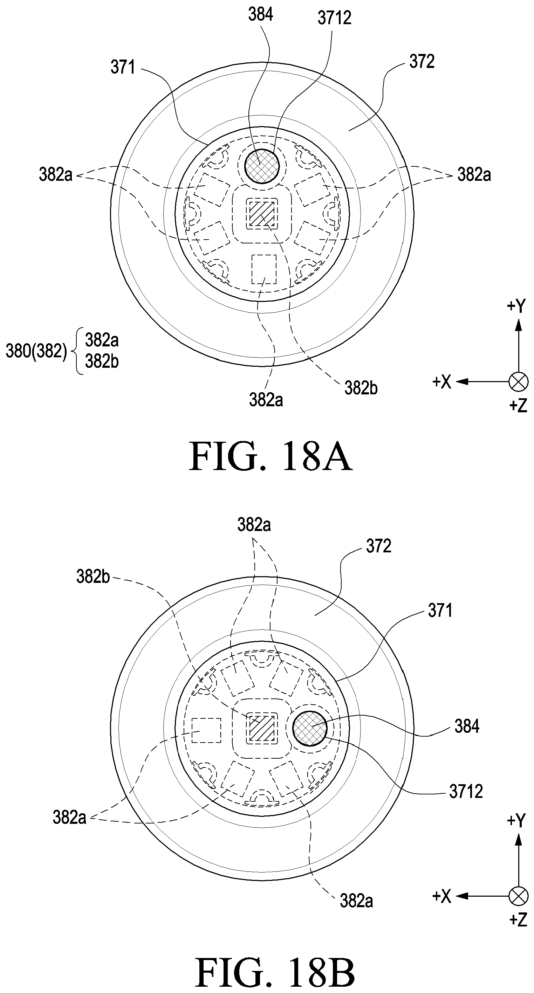

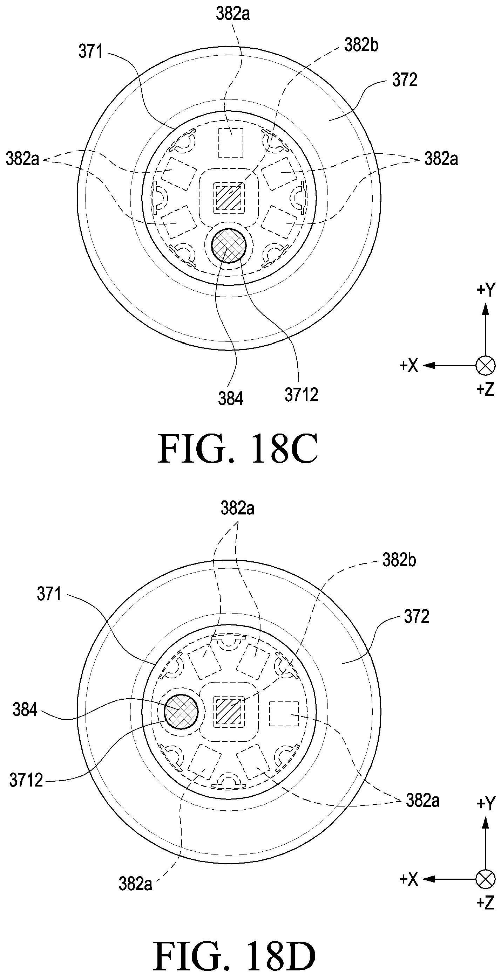

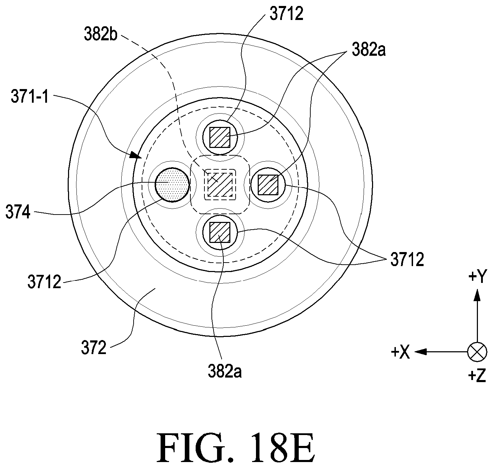

FIG. 18A is a plan view of a rear plate of a wearable electronic device according to an embodiment of the disclosure. FIG. 18B is a plan view of a rear plate of a wearable electronic device according to an embodiment of the disclosure. FIG. 18C is a plan view of a rear plate of a wearable electronic device according to an embodiment of the disclosure. FIG. 18D is a plan view of a rear plate of a wearable electronic device according to an embodiment of the disclosure. FIG. 18E is a plan view of a rear plate of a wearable electronic device according to an embodiment of the disclosure.

The embodiments of FIG. 18A to FIG. 18E do not technically contradict or conflict with the embodiments of FIG. 5 to FIG. 15C, and be combined with the embodiments of FIG. 5 to FIG. 15C.

The second rear plate 372 of the embodiments of FIG. 18A to FIG. 18E may refer to the rear plate 272 of the embodiments of FIG. 5 to FIG. 14. The optical sensors 282a and 282b and the temperature sensor 284 of the embodiments of FIG. 18A to FIG. 18E may refer to the optical sensors 282a and 282b and the temperature sensor 284 of the embodiments of FIG. 5 to FIG. 14.

A first rear plate 371 or 371-1 of the embodiments of FIG. 18A to FIG. 18E may have all the same configuration and feature as the configuration (e.g., the first coating layer 274a in FIG. 7B) and feature of the first rear plate 271 according to the embodiments of FIG. 5 to FIG. 14, except for the number and arrangement of the insert areas 3712 (e.g., the insert areas 2711 and 2712 in FIG. 7B). The embodiments of FIG. 18A to FIG. 18E will be described centering on the difference from the embodiments of FIG. 5 to FIG. 14, and overlapping descriptions thereof may be omitted below.

Referring to FIG. 18A to FIG. 18E, in an embodiment, the first rear plate 371 or 371-1 may include a single second insert area 3712 (e.g., the second insert area 2712 in FIG. 7A). According to an embodiment, the second insert area 3712 may be formed at a position beyond the central part of the rear plate 371 or 371-1. According to an embodiment, a temperature sensor 384 (e.g., a contact-type or a non-contact-type temperature sensor) may be disposed in the second insert area 3712. For example, the temperature sensor 384 may be a contact-type temperature sensor (e.g., the contact- type temperature sensor 284a in FIG. 10 and FIG. 11) such as a thermocouple, resistance temperature detectors (RTDs), or a bimetal temperature sensor (a bimetal thermometer), in addition to a thermistor. In an embodiment, the temperature sensor 384 may be a non-contact-type (or an optical-type) temperature sensor such as an infrared (IR) temperature sensor (thermopile).

Referring to FIG. 18A to FIG. 18E, according to an embodiment, at least one optical sensor 382a or 382b may be disposed on the first rear plate 371 or 371-1 without the application of an insert structure (e.g., the insert area 3712). Referring to FIG. 18A, FIG. 18B, FIG. 18C, and FIG. 18D, according to an embodiment, the light-emitting element 382b (e.g., an LED) may be disposed at the central part of the first rear plate 371 or 371-1. According to an embodiment, multiple (e.g., five) photoconversion elements 382a (e.g., photodiodes or phototransistors) may be arranged around the light-emitting element 382b. According to an embodiment, multiple (e.g., five) photoconversion elements 382a may be arranged to be spaced apart from each other, and for example, may be spaced a predetermined interval apart from each other. According to an embodiment, the photoconversion elements 382a and the light-emitting element 382b may be arranged on a first auxiliary circuit board (e.g., the first auxiliary circuit board 281 in FIG. 11) inside the first rear plate 371 or 371-1. Referring to FIG. 18E, in an embodiment, the first rear plate 371 or 371-1 may include multiple (e.g., four) second insert areas 3712. According to an embodiment, the photoconversion elements 382a may be respectively arranged in multiple (e.g., three) second insert areas 3712 other than the second insert area 3712 in which the temperature sensor 374 is disposed.

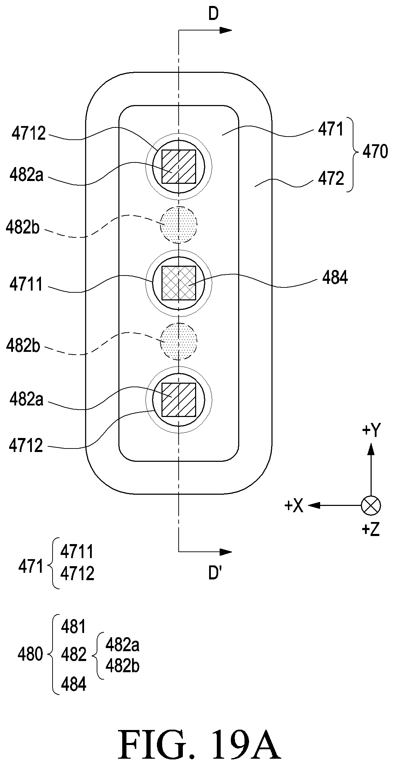

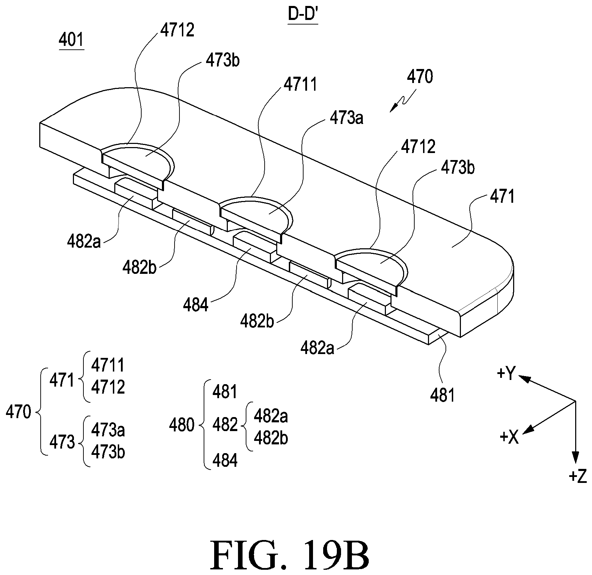

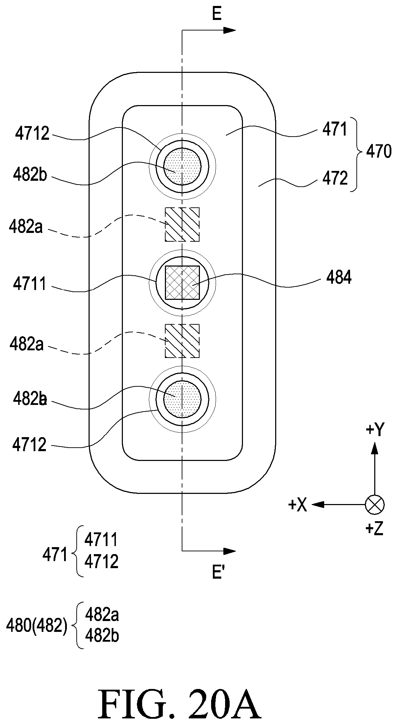

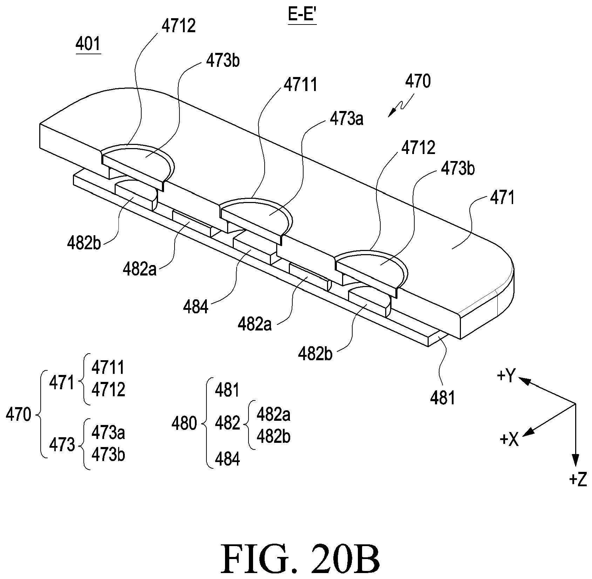

FIG. 19A is a plan view of a rear plate of a wearable electronic device according to an embodiment of the disclosure. FIG. 19B is a cross-sectional perspective view taken along line D-D' in FIG. 19A. FIG. 20A is a plan view of a rear plate of a wearable electronic device according to an embodiment of the disclosure. FIG. 20B is a cross-sectional perspective view taken along line E-E' in FIG. 20A.

The embodiments of FIG. 19A to FIG. 20B do not technically contradict or conflict with the embodiments of FIG. 5 to FIG. 15C, and be combined with the embodiments of FIG. 5 to FIG. 15C. The embodiments of FIG. 19A to FIG. 20B will be described centering on the difference from the embodiments of FIG. 5 to FIG. 14, and overlapping descriptions thereof may be omitted below.

Referring to FIG. 19A to FIG. 20B, in an embodiment, an electronic device 401 may include a first rear plate 471, a second rear plate 472, a cover member 473, and a sensor module 480. The sensor module 480 may include a first auxiliary circuit board 481, an optical sensor 482, and a temperature sensor 484. The second rear plate 472, the cover member 473, and the sensor module 480 of the embodiments of FIG. 19A to FIG. 20B may refer to the rear plate 272, the cover member 273, and the sensor module 280 of the embodiments of FIG. 5 to FIG. 14. The first rear plate 471 of the embodiments of FIG. 19A to FIG. 20B may have all the same configuration and feature as the configuration (e.g., the first coating layer 274a in FIG. 7B) and feature of the first rear plate 271 according to the embodiments of FIG. 5 to FIG. 14, except for the number and arrangement and the insert areas 4711 and 4712 (e.g., the insert areas 2711 and 2712 in FIG. 7B).

Referring to FIG. 19A to FIG. 20B, in an embodiment, the shape of the first rear plate 471 may be a quadrilateral shape. According to an embodiment, the second rear plate 472 disposed to surround the edge of the first rear plate 471 may have a quadrilateral shape. According to an embodiment, the quadrilateral shape of the first rear plate 471 and the second rear plate 472 may have corners chamfered as curved surfaces. In an embodiment, the first rear plate 471 may include a first insert area 4711 formed at the central part thereof, and multiple (e.g., two) second insert areas 4712 formed around (e.g., at both sides) of the first insert area 4711. In an embodiment, a temperature sensor 484 (e.g., a contact-type or a non-contact-type temperature sensor) may be disposed in the first insert area 4711.

According to an embodiment, the cover member may include a first cover member 473a (e.g., the first cover member 273a in FIG. 11) disposed in the first insert area 4711, and second cover members 473b (e.g., the second cover member 273b in FIG. 11) arranged in the second insert areas 4712.

Referring to FIG. 19A and FIG. 19B, in an embodiment, photoconversion elements 482a (e.g., photodiodes or phototransistors) may be arranged in the multiple (e.g., two) second insert areas 4712, respectively. In an embodiment, light-emitting elements 482b (e.g., LEDs) may be arranged in areas between the first insert area 4711 and the multiple (e.g., two) second insert areas 4712, respectively.

Referring to FIG. 20A and FIG. 20B, in an embodiment, light-emitting elements 482b may be arranged in the multiple (e.g., two) second insert areas 4712, respectively. In an embodiment, photoconversion elements 482a may be arranged in areas between the first insert area 4711 and the multiple (e.g., two) second insert areas 4712, respectively.

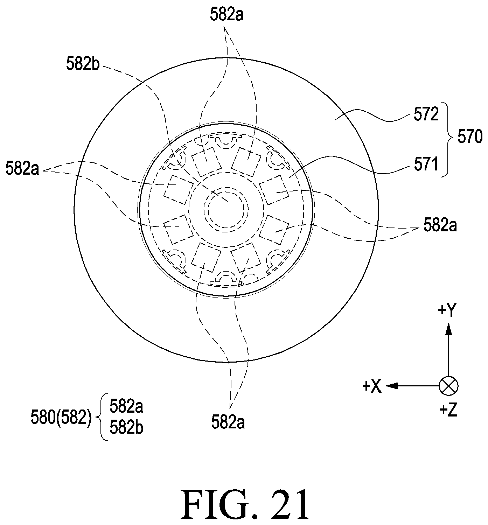

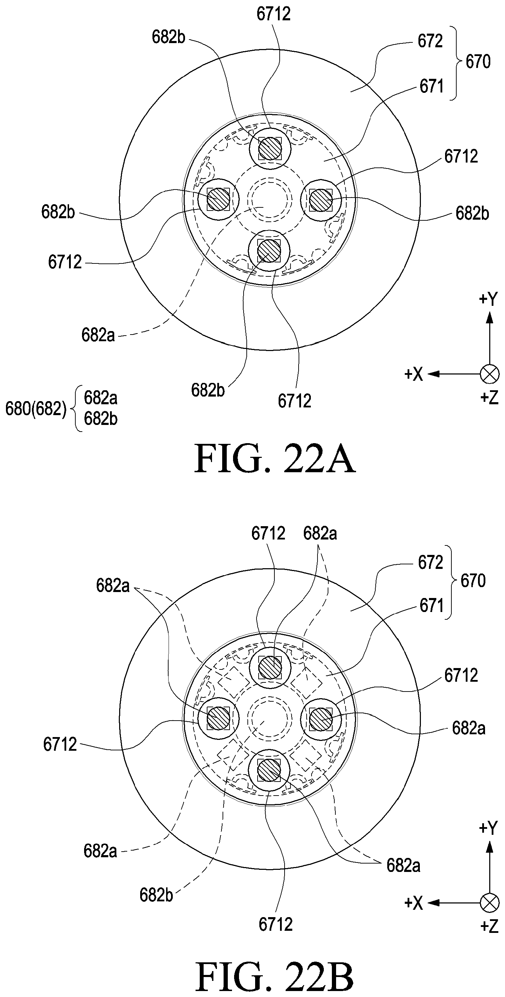

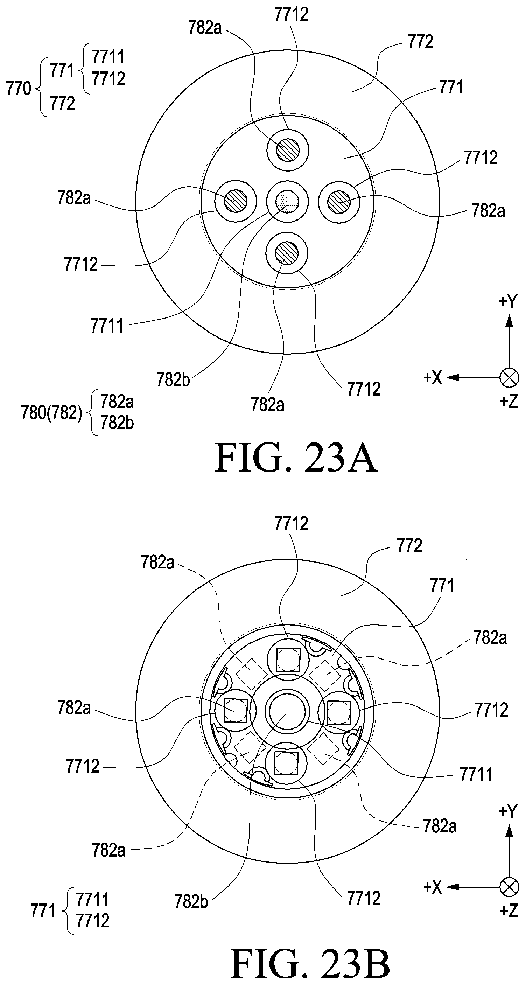

FIG. 21 is a plan view of a rear plate of a wearable electronic device according to an embodiment of the disclosure. FIG. 22A is a plan view of a rear plate of a wearable electronic device according to an embodiment of the disclosure. FIG. 22B is a plan view of a rear plate of a wearable electronic device according to an embodiment of the disclosure. FIG. 23A is a plan view of a rear plate of a wearable electronic device according to an embodiment of the disclosure. FIG. 23B is a plan view of a rear plate of a wearable electronic device according to an embodiment of the disclosure.

The embodiments of FIG. 21 to FIG. 23B do not technically contradict or conflict with the embodiments of FIG. 5 to FIG. 15C, and be combined with the embodiments of FIG. 5 to FIG. 15C. Differently from the embodiments of FIG. 5 to FIG. 20B, the embodiments of FIG. 21 to FIG. 23B may not include a temperature sensor. The embodiments of FIG. 21 to FIG. 23B will be described centering on the difference from the embodiments of FIG. 5 to FIG. 14, and overlapping descriptions thereof may be omitted below.

Second rear plates 572, 672, and 772 of rear plates 570, 670, and 770 of the embodiments of FIG. 21 to FIG. 23B may refer to the rear plate 272 of the embodiments of FIG. 5 to FIG. 14. Optical sensors 582, 682, and 782 of sensor modules 580, 680, and 780 of the embodiments of FIG. 18A to FIG. 18E may refer to the optical sensor 282 of the optical sensor module 280 of the embodiments of FIG. 5 to FIG. 14.

First rear plates 571, 671, and 771 of the rear plates 570, 670, and 770 of the embodiments of FIG. 21 to FIG. 23B may have all the same configuration and feature as the configuration (e.g., the first coating layer 274a in FIG. 7B) and feature of the first rear plate 271 according to the embodiments of FIG. 5 to FIG. 14, except for the presence of the insert areas 2711 and 2712 (see FIG. 7B) or the number and arrangement of the insert areas 2711 and 2712 of the embodiments of FIG. 5 to FIG. 14.

Referring to FIG. 21, insert areas (e.g., the insert areas 2711 and 2712 in FIG. 7B) may not be formed in the first rear plate 571. According to an embodiment, a light-emitting element 582b (e.g., an LED) may be disposed at the central part of the first rear plate 571. According to an embodiment, multiple (e.g., eight) photoconversion elements 582a (e.g., photodiodes or phototransistors) may be arranged around the light-emitting element 582b. According to an embodiment, the multiple (e.g., eight) photoconversion elements 582a may be arranged to be spaced apart from each other, and for example, may be spaced a predetermined interval apart from each other. According to an embodiment, the photoconversion elements 582a and the light-emitting element 582b may be arranged on a first auxiliary circuit board (e.g., the first auxiliary circuit board 281 in FIG. 11) inside the first rear plate 571.

Referring to FIG. 22A and FIG. 22B, in an embodiment, the first rear plate 671 may include multiple (e.g., four) second insert areas 6712. Referring to FIG. 22A, according to an embodiment, a photoconversion element 682a (e.g., a photodiode or a phototransistor) may be disposed at the central part of the first rear plate 671. According to an embodiment, light-emitting elements 682b (e.g., LEDs) may be arranged in the multiple (e.g., four) second insert areas 6712, respectively. Referring to FIG. 22B, according to an embodiment, a light-emitting element 682b (e.g., a LED) may be disposed at the central part of the first rear plate 671. According to an embodiment, photoconversion elements 682a (e.g., photodiodes or phototransistors) may be arranged in multiple (e.g., four) second insert areas 6712, respectively. According to an embodiment, photoconversion elements 682a (e.g., photodiodes or phototransistors) may be respectively arranged in areas between second insert areas 6712 adjacent to each other. According to an embodiment, the photoconversion elements 682a and the light-emitting element 682b may be arranged on a first auxiliary circuit board (e.g., the first auxiliary circuit board 281 in FIG. 11) inside the first rear plate 671.

Referring to FIG. 23A to FIG. 23B, in an embodiment, the first rear plate 771 may include a first insert area 7711 formed at the central part thereof, and multiple (e.g., four) second insert areas 7712 formed around the first insert area 7711. According to an embodiment, a light-emitting element 782b (e.g., an LED) may be disposed in the first insert area 7711. According to an embodiment, photoconversion elements 782a (e.g., photodiodes or phototransistors) may be arranged in multiple (e.g., four) second insert areas 7712, respectively.

Referring to FIG. 23B, according to an embodiment, photoconversion elements 782a (e.g., photodiodes or phototransistors) may be respectively arranged in areas between second insert areas 7712 adjacent to each other. According to an embodiment, the photoconversion elements 782a and the light-emitting element 782b may be arranged on a first auxiliary circuit board (e.g., the first auxiliary circuit board 281 in FIG. 11) inside the first rear plate 771.

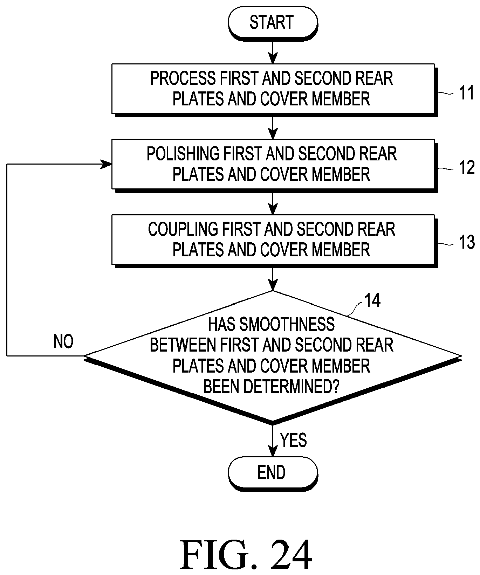

FIG. 24 is a flowchart of a method for manufacturing a rear plate of an electronic device according to an embodiment of the disclosure.

In the following embodiment, operations may also be sequentially performed, but may not be necessarily sequentially performed. For example, the order of operations may also be changed, and at least two operations may also be performed in parallel.

The manufacturing method in FIG. 24 may be applied to the first rear plates 271, 271-1, 271-2, 271-3, 371, 471, 571, 671, and 771, the second rear plates 272, 272-1, 272-2, 272-3, 372, 472, 572, 672, and 772, and the cover members 273 and 473 of the embodiments of FIG. 5 to FIG. 23B.

Referring to FIG. 24, in an embodiment of the disclosure, the manufacturing method may include an operation 11 for processing the first rear plate 271, 271-1, 271-2, 271-3, 371, 471, 571, 671, or 771, the second rear plate 272, 272-1, 272-2, 272-3, 372, 472, 572, 672, or 772, and the cover member 273 or 473 (or 'processing a first and a second rear plate and a cover member'). Here, for example, the processing of the operation 11 may include injection molding, sintering, and computer numerical control (CNC) processing.

According to an embodiment, the manufacturing method may include an operation 12 for polishing the first rear plate 271, 271-1, 271-2, 271-3, 371, 471, 571, 671, or 771, the second rear plate 272, 272-1, 272-2, 272-3, 372, 472 , 572, 672, or 772, and the cover member 273 or 473 (or 'polishing the first and the second rear plate and the cover member'). Through the polishing of the operation 12, the detailed dimensions of the curved surface parts of the first rear plate 271, 271-1, 271-2, 271-3, 371, 471, 571, 671, or 771, the second rear plate 272, 272-1, 272-2, 272-3, 372, 472 , 572, 672, or 772, and the cover member 273 or 473 may be adjusted.

According to an embodiment, the manufacturing method may include an operation 13 for coupling the first rear plate 271, 271-1, 271-2, 271-3, 371, 471, 571, 671, or 771, the second rear plate 272, 272-1, 272-2, 272-3, 372, 472 , 572, 672, or 772, and the cover member 273 or 473 (or 'coupling the first and the second rear plate and the cover member'). For example, in the operation 13, an adhesive or an adhesive member such as double-sided tape may be used.

According to an embodiment, the manufacturing method may include an operation 14 for determining the smoothness between the first rear plate 271, 271-1, 271-2, 271-3, 371, 471, 571, 671, or 771, the second rear plate 272, 272-1, 272-2, 272-3, 372, 472 , 572, 672, or 772, and the cover member 273 or 473 (or 'determining the smoothness between the first and the second rear plate and the cover member'). In the operation 14, after determining a step of the coupled part between the first rear plate 271, 271-1, 271-2, 271-3, 371, 471, 571, 671, or 771 and the second rear plate 272, 272-1, 272-2, 272-3, 372, 472 , 572, 672, or 772, or a step of the coupled part between the first rear plate 271, 271-1, 271-2, 271-3, 371, 471, 571, 671, or 771 and the cover member 273 or 473, the portion at which the step is generated or the whole portion thereof may be polished to remove the step. In the operation 14, in case the smoothness is greater than a reference value, the manufacturing method may be completed (or terminated). In the operation 14, in case the smoothness is less than the reference value, after returning to the above-described operation 12, operations 12 to 14 may be repeated.

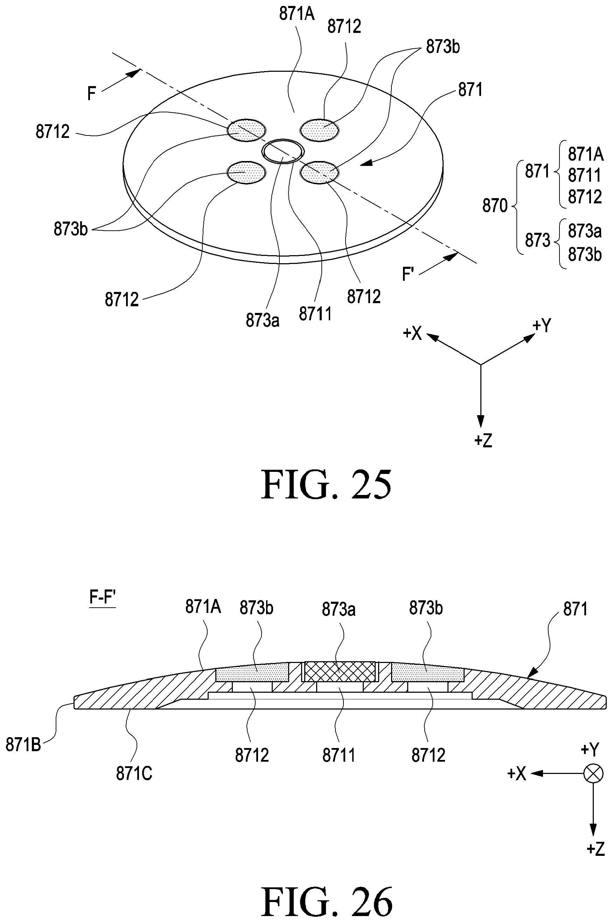

FIG. 25 is a perspective view of a rear plate of a wearable electronic device according to an embodiment of the disclosure. FIG. 26 is a cross-sectional view taken along line F-F' in FIG. 8.

Referring to FIG. 25 and FIG. 26, in an embodiment, a wearable electronic device (e.g., the electronic device 101 in FIG. 1 and/or the wearable electronic device 200 in FIG. 2 to FIG. 4) may include a rear plate 871 including multiple insert areas 8711 and 8712, and multiple cover members 873 arranged in the insert areas 8711 and 8712. According to an embodiment, the rear plate 871 may be referred to as a single plate member in which a first rear plate (e.g., the first rear plate 271, 271-1, 271-2, 271-3, 371, 471, 571, 671, or 771) and a second rear plate (e.g., the second rear plate 272, 272-1, 272- 2, 272-3, 372, 472, 572, 672, or 772) are integrally formed or manufactured.

The rear plate 871 and the cover members 873 in FIG. 25 and FIG. 26 may be entirely or partially identical to the first rear plate 271, 271-1, 271-2, 271-3, 371, 471, 571, 671, or 771 and the cover member 273 or 473 described with reference to FIG. 5 to FIG. 24. As an example, the rear plate 871 may further include a first coating area (e.g., the first coating area 274a in FIG. 7B) formed on at least one of the side surfaces of the insert areas 8711 and 8712, and the cover members 873 may include a second coating area (e.g., the second coating area 274b in FIG. 11). Here, the second coating area may be formed on at least a part of the side surfaces of the cover members 873, which faces a side surface of the insert areas 8711 and 8712. The configuration of the rear plate 871 and the cover members 873 in FIG. 25 and FIG. 26 may be entirely or partially identical to the configuration of the first rear plate 271, 271-1, 271-2, 271-3, 371, 471, 571, 671, or 771 and the cover member 273 or 473 described with reference to FIG. 5 to FIG. 24, in addition to the above-described first coating area 274a and/or second coating area 274b, and overlapping descriptions may be omitted below.

In an embodiment, the rear plate 871 may include a (1-1)th surface 871A facing the outside (e.g., the -Z-axis direction) of a wearable electronic device (e.g., the wearable electronic device 200 in FIG. 2 to FIG. 4), a (1-2)th surface 871B oriented in a direction opposite to the (1-1)th surface 871A, and a first side surface 871C for connecting the (1-1)th surface 871A and the (1-2)th surface 871B. For example, the (1-1)th surface 871A of the rear plate 871 may be formed to include a flat surface and/or a curved surface. In an embodiment, the rear plate 871 may include multiple insert areas 8711 and 8712 in which the cover members 873 are arranged. According to an embodiment, the multiple insert areas 8711 and 8712 may be formed through the portion from the (1-1)th surface 871A to the (1-2)th surface 871B, in the rear plate 871.

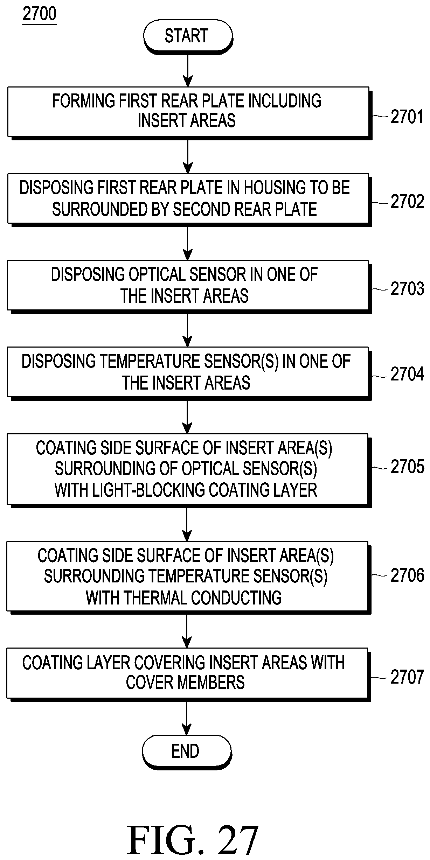

FIG. 27 is a flow diagram illustrating a method of assembling a wearable electronic device according to an embodiment of the disclosure.

With reference to FIG. 27, in an embodiment, a method 2700 may be provided for assembling a wearable electronic device 101, 200 and/or 401, such as the wearable electronic device described herein, that is to be worn next to skin (i.e., a wrist) of a user. The method 2700 may include forming a first rear plate 271, 271-1, 271-2, 471, 571, 671 and/or 771 that includes insert areas 2711, 2712, 47111, 4712, and/or 8711, 8712 formed as recesses on a surface of the first rear plate (2701) and disposing the first rear plate in a housing to be surrounded by a second rear plate 272, 372, 472, 572, 672 and/or 772 (2702). The method 2700 may further include disposing an optical sensor 282, 382a, 382b, 482, 582, 682 and/or 782 in at least one of the insert areas (2703), disposing a temperature sensor 284, 384 and/or 484 in another at least one of the insert areas (2704), coating a side surface of the at least one of the insert areas in which the optical sensor is disposed with a light-blocking coating layer (2705) and coating a side surface of the at least one of the insert areas in which the temperature sensor is disposed with a thermal conductive coating layer (2706). The method 2700 may further include covering the insert areas with cover members 273, 473 (2707).

In an embodiment, the forming of the first rear plate (2701) may include forming the first rear plate to be flat. According to an embodiment, the cover members may be flat whereby the first rear plate and the cover members form a flat surface.

In an embodiment, the forming of the first rear plate of (2701) may include forming the first rear plate to be curved. According to an embodiment, the cover members maybe curved whereby the first rear plate and the cover members form a curved surface.

In an embodiment, the forming of the first rear plate (2701) may include forming a first insert area in a central region of the first rear plate and forming second insert areas around the first insert area (27011), the disposing of the temperature sensor (2704) may include disposing a single temperature sensor in the first insert area (27041) and the disposing of the optical sensor (2703) may include disposing optical sensors in the second insert areas (27031).

In addition, as described above though not shown in FIG. 27, the method 2700 may also include disposing the optical sensor on a first auxiliary circuit board, disposing a second auxiliary circuit board between the first rear plate and the first auxiliary circuit board, disposing the temperature sensor as a first temperature sensor on a surface of the second auxiliary circuit board facing the cover members and, disposing the temperature sensor as a second temperature sensor on a surface of the first auxiliary board facing the second auxiliary board, and/or configuring an electrode structure on the rear plate to measure biometric information.

The above-described descriptions with reference to the accompanying drawings may be provided to assist in a comprehensive understanding for variously implementing the disclosure defined by the claims. The specific embodiments disclosed in the above-described descriptions include various specific details for helping understanding, but may be regarded as one of embodiments. In addition, descriptions of well-known functions and configurations may be omitted for clarity and conciseness.

The terms and words used in the above-described descriptions and the claims are limited to the bibliographical meanings, and may be used to clearly and consistently describe embodiments disclosed in the document. Accordingly, it should be obvious to a person skilled in the art that the above-described descriptions for various implements of the disclosure are provided for an explanation purpose only and not for the purpose that the claims are limited by the disclosure.

The singular forms "a", "an", and "the" should be understood to include the plural forms as well, unless the context clearly indicates otherwise. Therefore, for example, "a surface of an element" may mean including one or more of surfaces of the element.

The electronic device (or the wearable electronic device) according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program) including one or more instructions that are stored in a storage medium (e.g., internal memory or external memory) that is readable by a machine (e.g., the electronic device 101 or the wearable electronic device 200). For example, a processor (e.g., the processor) of the machine (e.g., the electronic device 101 or the wearable electronic device 200) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. A wearable electronic device (101, 200, 401) comprising:
a housing (210);
a display (220) disposed on a first surface of the housing;
a rear plate (207, 270) disposed on a second surface of the housing opposite the first surface;
and
a sensor module (160, 211, 280) disposed in the housing,
wherein the rear plate comprises a first rear plate (271) comprising insert areas (2711, 2712), a second rear plate (272) configured to surround at least a part of the first rear plate and cover members (273) disposed to close the insert areas,
the sensor module comprises at least one optical sensor and at least one temperature sensor (284) disposed in spaces surrounded by the insert areas and the cover members, and
a first coating area (274a) comprising at least one of a thermal conductive coating layer or a light-blocking coating layer is formed on at least a part of at least one of side surfaces of the insert areas,
the wearable electronic device (101, 200, 401) further comprising:
a first auxiliary circuit board (281) on which the optical sensor is disposed; and
a second auxiliary circuit board (283) disposed between the first rear plate and the first auxiliary circuit board
wherein the temperature sensor comprises:
a first temperature sensor (2841a) disposed on a surface of the second auxiliary circuit board; and
a second temperature sensor (2842a) disposed on a surface of the first auxiliary circuit board.

2. The wearable electronic device of claim 1, wherein a second coating area (274b) comprising at least one of a thermal conductive coating layer or a light-blocking coating layer is formed on at least a part of at least one of side surfaces of the cover members.

3. The wearable electronic device of claim 1 or 2, wherein the insert areas comprise:
a first insert area (2711) formed at a central part of the first rear plate and at least one second insert area (2712) formed around the first insert area.

4. The wearable electronic device of claim 3, wherein:
the temperature sensor is disposed in the first insert area, and
the optical sensor is disposed in the at least one second insert area.

5. The wearable electronic device of claim 3 or 4, wherein the cover members comprise:
a first cover member (273a) disposed on the first insert area; and
at least one second cover member (273b) disposed on the at least one second insert area.

6. The wearable electronic device of claim 5, wherein:
the temperature sensor is configured to measure a temperature based on heat conducted through the rear plate, and
the second coating area (274b) is formed on a side surface of the first cover member and comprises a thermal conductive coating layer.

7. The wearable electronic device of claim 5 or 6, wherein:
the temperature sensor is configured to measure a temperature based on infrared rays transmitted through the rear plate, and
the second coating area (274b) is formed on a side surface of the at least one second cover member and comprises a light-blocking coating layer.

8. The wearable electronic device of any one of claim 5 to 7, wherein:
the optical sensor comprises at least one light-emitting element (282b) and at least one photoelectric conversion element (282a), and
the at least one second cover member is configured to perform at least one of selective transmitting of light of a specific wavelength or selective blocking of light of a specific wavelength.

9. The wearable electronic device of claim 1, wherein:
the rear plate is formed to be at least partially opaque, and
the sensor module comprises an electrode structure configured to measure biometric information of a user on an inner surface of the rear plate.

10. The wearable electronic device of claim 9, further comprising:
a printed circuit board (250) disposed between the display and the first auxiliary circuit board;
and
a bridge connector (287) disposed between the rear plate and the sensor module and electrically connected to the electrode structure and the printed circuit board.

11. The wearable electronic device of any one of claim 1 to 10, wherein each of the insert areas comprises:
a first portion (2711-1, 2712-1) formed to surround a side surface of each of the cover members; and
a second portion (2711-2, 2712-2) extending from the first portion,
wherein:
the optical sensor or the temperature sensor is disposed inside the second portion, and
a width of the first portion is greater than a width of the second portion.

12. The wearable electronic device of any one of claim 1 to 11, wherein the first rear plate and the cover members form a flat surface or a curved surface.

13. The wearable electronic device of claim 1, further comprising a processor (120) communicably coupled to the first and second temperature sensors, wherein the processor is configured to: derive additional biometric information based on the temperature readings from both the first temperature sensor and the second temperature sensor.

14. The wearable electronic device of claim 13, wherein the additional biometric information is a heart temperature.

## Patentansprüche

1. Tragbare elektronische Vorrichtung (101, 200, 401), umfassend:
ein Gehäuse (210);
eine Anzeige (220), die auf einer ersten Oberfläche des Gehäuses angeordnet ist;
eine Rückplatte (207, 270), die auf einer der ersten Oberfläche gegenüberliegenden zweiten Oberfläche des Gehäuses angeordnet ist; und
ein Sensormodul (160, 211, 280), das im Gehäuse angeordnet ist,
wobei die Rückplatte eine erste Rückplatte (271) mit Einsteckbereichen (2711, 2712), eine zweite Rückplatte (272), die dazu konfiguriert ist, zumindest einen Teil der ersten Rückplatte zu umgeben, und Abdeckelemente (273) umfasst, die so angeordnet sind, dass sie die Einsteckbereiche verschließen,
das Sensormodul mindestens einen optischen Sensor und mindestens einen Temperatursensor (284) umfasst, die in von den Einsteckbereichen und den Abdeckelementen umschlossenen Räumen angeordnet sind, und
auf mindestens einem Teil mindestens einer der Seitenflächen der Einsteckbereiche ein erster Beschichtungsbereich (274a) ausgebildet ist, der mindestens eine von einer wärmeleitenden Beschichtungsschicht und einer lichtblockierenden Beschichtungsschicht umfasst,
die tragbare elektronische Vorrichtung (101, 200, 401) ferner Folgendes umfasst:
eine erste Hilfsschaltplatte (281), auf der der optische Sensor angeordnet ist; und
eine zweite Hilfsschaltplatte (283), die zwischen der ersten Rückplatte und der ersten Hilfsschaltplatte angeordnet ist,
wobei der Temperatursensor Folgendes umfasst:
einen ersten Temperatursensor (2841a), der auf einer Oberfläche der zweiten Hilfsschaltplatte angeordnet ist; und
einen zweiten Temperatursensor (2842a), der auf einer Oberfläche der ersten Hilfsschaltplatte angeordnet ist.

2. Tragbare elektronische Vorrichtung nach Anspruch 1, wobei auf mindestens einem Teil mindestens einer der Seitenflächen der Abdeckelemente ein zweiter Beschichtungsbereich (274b) ausgebildet ist, der mindestens eine von einer wärmeleitenden Beschichtungsschicht und einer lichtblockierenden Beschichtungsschicht umfasst.

3. Tragbare elektronische Vorrichtung nach Anspruch 1 oder 2, wobei die Einsteckbereiche Folgendes umfassen:
einen ersten Einsteckbereich (2711), der an einem zentralen Teil der ersten Rückplatte ausgebildet ist, und mindestens einen zweiten Einsteckbereich (2712), der um den ersten Einsteckbereich herum ausgebildet ist.

4. Tragbare elektronische Vorrichtung nach Anspruch 3, wobei:
der Temperatursensor im ersten Einsteckbereich angeordnet ist und
der optische Sensor in dem mindestens einen zweiten Einsteckbereich angeordnet ist.

5. Tragbare elektronische Vorrichtung nach Anspruch 3 oder 4, wobei die Abdeckelemente Folgendes umfassen:
ein erstes Abdeckelement (273a), das auf dem ersten Einsteckbereich angeordnet ist;
und
mindestens ein zweites Abdeckelement (273b), das auf dem mindestens einen zweiten Einsteckbereich angeordnet ist.

6. Tragbare elektronische Vorrichtung nach Anspruch 5, wobei:
der Temperatursensor dazu konfiguriert ist, eine Temperatur auf der Grundlage der durch die Rückplatte geleiteten Wärme zu messen, und
der zweite Beschichtungsbereich (274b) auf einer Seitenfläche des ersten Abdeckelements ausgebildet ist und eine wärmeleitende Beschichtungsschicht umfasst.

7. Tragbare elektronische Vorrichtung nach Anspruch 5 oder 6, wobei:
der Temperatursensor dazu konfiguriert ist, eine Temperatur auf der Grundlage von durch die Rückplatte übertragenen Infrarotstrahlen zu messen, und
der zweite Beschichtungsbereich (274b) auf einer Seitenfläche des mindestens einen zweiten Abdeckelements ausgebildet ist und eine lichtblockierende Beschichtungsschicht umfasst.

8. Tragbare elektronische Vorrichtung nach einem der Ansprüche 5 bis 7, wobei:
der optische Sensor mindestens ein lichtemittierendes Element (282b) und mindestens ein photoelektrisches Umwandlungselement (282a) umfasst, und
das mindestens eine zweite Abdeckelement dazu konfiguriert ist, mindestens eines von selektivem Durchlassen von Licht einer bestimmten Wellenlänge und selektivem Blockieren von Licht einer bestimmten Wellenlänge durchzuführen.

9. Tragbare elektronische Vorrichtung nach Anspruch 1, wobei:
die Rückplatte zumindest teilweise opak ausgebildet ist, und
das Sensormodul eine Elektrodenstruktur umfasst, die dazu konfiguriert ist, biometrische Informationen eines Benutzers auf einer Innenfläche der Rückplatte zu messen.

10. Tragbare elektronische Vorrichtung nach Anspruch 9, umfassend ferner:
eine Leiterplatte (250), die zwischen der Anzeige und der ersten Hilfsschaltplatte angeordnet ist; und
einen Brückenverbinder (287), der zwischen der Rückplatte und dem Sensormodul angeordnet und elektrisch mit der Elektrodenstruktur und der Leiterplatte verbunden ist.

11. Tragbare elektronische Vorrichtung nach einem der Ansprüche 1 bis 10, wobei jeder der Einsteckbereiche Folgendes umfasst:
einen ersten Abschnitt (2711-1, 2712-1), der so ausgebildet ist, dass er eine Seitenfläche jedes der Abdeckelemente umgibt; und
einen zweiten Abschnitt (2711-2, 2712-2), der sich vom ersten Abschnitt erstreckt,
wobei:
der optische Sensor oder der Temperatursensor innerhalb des zweiten Abschnitts angeordnet ist und
eine Breite des ersten Abschnitts größer ist als eine Breite des zweiten Abschnitts.

12. Tragbare elektronische Vorrichtung nach einem der Ansprüche 1 bis 11, wobei die erste Rückplatte und die Abdeckelemente eine ebene Oberfläche oder eine gekrümmte Oberfläche bilden.

13. Tragbare elektronische Vorrichtung nach Anspruch 1, umfassend ferner einen Prozessor (120), der kommunikativ mit dem ersten und dem zweiten Temperatursensor gekoppelt ist, wobei der Prozessor dazu konfiguriert ist: zusätzliche biometrische Informationen auf der Grundlage der Temperaturmesswerte sowohl vom ersten Temperatursensor als auch vom zweiten Temperatursensor abzuleiten.

14. Tragbare elektronische Vorrichtung nach Anspruch 13, wobei die zusätzlichen biometrischen Informationen eine Herztemperatur sind.

## Revendications

1. Dispositif électronique à porter (101, 200, 401) comprenant:
un boîtier (210);
un affichage (220) disposé sur une première surface du boîtier;
une plaque arrière (207, 270) disposée sur une seconde surface du boîtier opposée à la première surface; et
un module de capteur (160, 211, 280) disposé dans le boîtier,
dans lequel la plaque arrière comprend une première plaque arrière (271) comprenant des zones d'insertion (2711, 2712), une seconde plaque arrière (272) configurée pour entourer au moins une partie de la première plaque arrière et des éléments de recouvrement (273) disposés pour fermer les zones d'insertion,
le module de capteur comprend au moins un capteur optique et au moins un capteur de température (284) disposés dans des espaces entourés par les zones d'insertion et les éléments de recouvrement, et
une première zone de revêtement (274a) comprenant au moins une parmi une couche de revêtement thermoconductrice ou une couche de revêtement bloquant la lumière est formée sur au moins une partie d'au moins une parmi les surfaces latérales des zones d'insertion,
le dispositif électronique à porter (101, 200, 401) comprenant en outre:
une première carte de circuit auxiliaire (281) sur laquelle est disposé le capteur optique;
et
une seconde carte de circuit auxiliaire (283) disposée entre la première plaque arrière et la première carte de circuit auxiliaire,
dans lequel le capteur de température comprend:
un premier capteur de température (2841a) disposé sur une surface de la seconde carte de circuit auxiliaire; et
un second capteur de température (2842a) disposé sur une surface de la première carte de circuit auxiliaire.

2. Dispositif électronique à porter selon la revendication 1, dans lequel une seconde zone de revêtement (274b) comprenant au moins une parmi une couche de revêtement thermoconductrice ou une couche de revêtement bloquant la lumière est formée sur au moins une partie d'au moins une parmi les surfaces latérales des éléments de recouvrement.

3. Dispositif électronique à porter selon la revendication 1 ou 2, dans lequel les zones d'insertion comprennent:
une première zone d'insertion (2711) formée dans une partie centrale de la première plaque arrière et au moins une seconde zone d'insertion (2712) formée autour de la première zone d'insertion.

4. Dispositif électronique à porter selon la revendication 3, dans lequel:
le capteur de température est disposé dans la première zone d'insertion, et
le capteur optique est disposé dans l'au moins une seconde zone d'insertion.

5. Dispositif électronique à porter selon la revendication 3 ou 4, dans lequel les éléments de recouvrement comprennent:
un premier élément de recouvrement (273a) disposé sur la première zone d'insertion; et
au moins un second élément de recouvrement (273b) disposé sur l'au moins une seconde zone d'insertion.

6. Dispositif électronique à porter selon la revendication 5, dans lequel:
le capteur de température est configuré pour mesurer une température sur la base de la chaleur conduite à travers la plaque arrière, et
la seconde zone de revêtement (274b) est formée sur une surface latérale du premier élément de recouvrement et comprend une couche de revêtement thermoconductrice.

7. Dispositif électronique à porter selon la revendication 5 ou 6, dans lequel:
le capteur de température est configuré pour mesurer une température sur la base de rayons infrarouges transmis à travers la plaque arrière, et
la seconde zone de revêtement (274b) est formée sur une surface latérale dudit au moins un second élément de recouvrement et comprend une couche de revêtement bloquant la lumière.

8. Dispositif électronique à porter selon l'une quelconque des revendications 5 à 7, dans lequel:
le capteur optique comprend au moins un élément émetteur de lumière (282b) et au moins un élément de conversion photoélectrique (282a), et
l'au moins un second élément de recouvrement est configuré pour effectuer au moins l'un parmi une transmission sélective de lumière d'une longueur d'onde spécifique ou un blocage sélectif de lumière d'une longueur d'onde spécifique.

9. Dispositif électronique à porter selon la revendication 1, dans lequel:
la plaque arrière est formée de manière à être au moins partiellement opaque, et
le module de capteur comprend une structure d'électrode configurée pour mesurer des informations biométriques d'un utilisateur sur une surface interne de la plaque arrière.

10. Dispositif électronique à porter selon la revendication 9, comprenant en outre:
une carte de circuit imprimé (250) disposée entre l'affichage et la première carte de circuit auxiliaire; et
un connecteur de pont (287) disposé entre la plaque arrière et le module de capteur et connecté électriquement à la structure d'électrode et à la carte de circuit imprimé.

11. Dispositif électronique à porter selon l'une quelconque des revendications 1 à 10, dans lequel chacune des zones d'insertion comprend:
une première partie (2711-1, 2712-1) formée pour entourer une surface latérale de chacun des éléments de recouvrement; et
une seconde partie (2711-2, 2712-2) s'étendant à partir de la première partie,
dans lequel:
le capteur optique ou le capteur de température est disposé à l'intérieur de la seconde partie, et
une largeur de la première partie est supérieure à une largeur de la seconde partie.

12. Dispositif électronique à porter selon l'une quelconque des revendications 1 à 11, dans lequel la première plaque arrière et les éléments de recouvrement forment une surface plane ou une surface courbe.

13. Dispositif électronique à porter selon la revendication 1, comprenant en outre un processeur (120) couplé en communication aux premier et second capteurs de température, dans lequel le processeur est configuré pour: déduire des informations biométriques supplémentaires sur la base des relevés de température provenant à la fois du premier capteur de température et du second capteur de température.

14. Dispositif électronique à porter selon la revendication 13, dans lequel les informations biométriques supplémentaires sont une température cardiaque.
